# EUROPEAN PATENT APPLICATION

(11) **EP 2 650 002 A1**
(43) Date of publication of application: **16.10.2013**
(21) Application number: 11846843.8
(22) Date of filing: 07.12.2011
(51) Int. Cl.: A61K 35/74, A61P 3/04, A61P 3/10, A61P 37/02

(54) **BIFIDOBACTERIUM CECT 7765 AND USE THEREOF IN THE PREVENTION AND/OR TREATMENT OF EXCESS WEIGHT, OBESITY AND RELATED PATHOLOGIES**

(30) Priority: 07.12.2010 ES 201031811
(71) Applicant: Consejo Superior de Investigaciones Científicas C.S.I.C., 28006 Madrid (ES)
(72) Inventor: SANZ HERRANZ, Yolanda, E - 28006 Madrid (ES); ARLETTE SANTACRUZ, Yolanda, E - 28006 Madrid (ES); GAUFFIN, Paola, E - 28006 Madrid (ES)
(74) Representative: ABG Patentes, S.L.
(86) International application number: PCT/ES2011/070838
(87) International publication number: WO 2012/076739

(57) **Abstract**

The present invention relates to the *Bifidobacterium* CECT 7765 strain, to its cell components, metabolites, and secreted molecules, to the combinations thereof with other microorganisms, and to compositions comprising the aforementioned products, as well as to the use of a strain of the *Bifidobacterium pseudocatenulatum* species, or to using the CECT 7765 strain for the prevention and/or treatment of obesity, overweight, hyperglycemia and diabetes, preferably type 2 diabetes mellitus, hepatic steatosis or fatty liver, dyslipidemia, metabolic syndrome, immune system dysfunction associated with obesity and overweight; and an unbalanced composition of the intestinal microbiota associated with obesity and overweight.

## Description

The present invention is comprised in the field of the therapeutic activity of pharmaceutical compositions or preparations, and in the field of food. Specifically, the present invention relates to the *Bifidobacterium pseudocatenulatum* CECT 7765 strain, to its cell components, metabolites, and secreted molecules, to the combinations thereof with other microorganisms, and to compositions comprising the aforementioned products, as well as to using a strain of the *B. pseudocatenulatum* species or to using the CECT 7765 strain for the prevention and/or treatment of obesity, overweight, hyperglycemia and diabetes, preferably type 2 diabetes mellitus, hepatic steatosis or fatty liver, dyslipidemia, metabolic syndrome, immune system dysfunction associated with obesity and overweight, and an unbalanced composition of the intestinal microbiota associated with obesity and overweight.

### Prior State of the Art

Overweight and obesity are today one of the main public health problems due to their growing prevalence and co-morbilities. These include, for example, metabolic syndrome, hypertension, dyslipidemia, diabetes, cardiovascular diseases, atherosclerosis, hepatic steatosis or fatty liver and some types of cancer.

Obesity occurs as a result of a positive and prolonged imbalance between consumption and energy expenditure, which entails an excessive increase of body fat. Peptides and hormones synthesized by the neuroendocrine system which allow communication between different peripheral tissues and organs and the central nervous system which, overall, contribute to regulating body weight are involved in energy balance control. Signals from adipose tissue (leptin) and the pancreas (insulin) are fundamental in long-term consumption control (Konturek et al., 2004. J Physiol Pharmacol., 55: 137-154). Insulin is the most important hormone in regulating the proper working of adipose tissue and the accumulation of triglycerides therein and in glucose uptake. Fat is stored in normal insulin-sensitive adipose tissue, as a response to insulin and other hormones (leptin), by means of stimulating lipoprotein lipase and inhibiting lipolysis. However, the excessive accumulation of fatty acids in adipose tissue associated with obesity reduces insulin sensitivity, which diverts the accumulation of free fatty acids in the form of triglycerides to several organs and tissues (liver, muscle, heart, etc.) and causes alterations in the leptin production or sensitivity and increases proinflammatory cytokine synthesis, which leads to a higher risk of developing associated diseases (metabolic syndrome, cardiovascular diseases, etc). Insulin signaling is also essential in the central nervous system for energy balance control and glucose homeostasis, and it is dependent on its interaction with other regulatory factors, such as leptin, which act together as anorexigenic factors, reducing consumption (Gerozissis K., 2004. Eur J Pharmacol., 490(1-3): 59-70). Leptin is a hormone/adipokine synthesized primarily by adipose tissue and, to a lesser extent, by other tissues such as the stomach, and secretion thereof is stimulated by insulin. At the level of the central nervous system, leptin suppresses appetite, increases energy expenditure and is involved in vital processes such as the pancreatic β-cell function, favoring insulin secretion (La Cava A, Matarese G. The weight of leptin in immunity. Nat Rev Immunol. 2004 May;4(5):371-9). At the peripheral level, leptin acts by reducing fatty acid and triglyceride synthesis and by increasing lipid oxidation. Nevertheless, in obese subjects peripheral concentrations of this adipokine are abnormally high and adipokine resistance develops. In addition to being a marker for metabolic disorders, high leptin concentrations in obese subjects may modify the immune response and contribute to the condition of obesity-associated inflammation.

Obesity is also considered a condition of mild chronic inflammation, characterized by high production of cytokines, adipokines and other proinflammatory proteins in adipose tissue and at the systemic level which contribute to metabolic disorders such as type 2 diabetes mellitus that these individuals may permanently suffer from (Tilg and Moschen, 2006. Nat Rev Immunol., 6: 772-783). The inflammatory factors related to obesity and metabolic disorders particularly include the proinflammatory cytokines TNF-α and IL6, and the macrophage chemoattractant protein MCP1. In particular, TNF-α reduces the expression of genes involved in the action of insulin (for example its receptor gene expression), attenuates insulin signaling and inhibits the action of the insulin-stimulated lipoprotein lipase. MCP1 favors the infiltration of macrophages into the adipose tissue associated with weight gain, which contributes to the increased production of proinflammatory cytokines (TNF-α) and to the development of insulin resistance and hepatic steatosis. The function of proinflammatory cytokines in this process has also been demonstrated by means of using anti-TNF-α antibody-based drugs for improving pathologies such as steatosis, insulin resistance and type 2 diabetes mellitus (Tilg and Moschen, 2006. Nat Rev Immunol., 6: 772-783).

Obesity is also characterized by alterations in the functions of different immune system cells, such as macrophages, dendritic cells and T-cells, associated with deficiencies in the defense against pathogens and other antigens and with a higher risk of post-operatory infections and complications. Adipose tissue macrophages have a lower phagocytic capacity and reduced respiratory burst, which are processes involved in the response of the innate immune system against infectious agents (Zhou et al., 2009. Proc Natl Acad Sci U S A, 106(26):10740-5.). Furthermore, dendritic cells have a disrupted capacity to stimulate T-cells, which are involved in the adaptive immune response responsible, for example, for producing antibodies in vaccination and for the memory T-cell response in cases of infection (Karlsson et al., 2010. J Immunol., 184:3127-33).

Social changes associated with the regular increase of the consumption of foods with a high energy load and low physical activity are considered to be the main causes of the increased incidence of obesity worldwide. Nevertheless, traditional treatments based on low-calorie diets and increased physical activity show reduced efficacy in obesity control and, generally, lead to limited and temporary weight lost. The use of pharmacological strategies has not been satisfactory either because it entails side effects. Consequently, new intervention strategies which improve treatment and enable preventing these pathologies are still sought.

The microbiota colonizing the human intestine is considered a new factor involved in obesity and associated diseases through its capacity to regulate metabolic and immunological functions of the individual (Sanz et al., 2010. Proc Nutr Soc., 14: 1-8.). The detection of alterations in the composition of the microbiota associated with obesity has also led to proposing the intentional manipulation of the intestinal ecosystem as an alternative for controlling obesity (Ley et al., 2006. Nature, 444: 1022-1023; Nadal et al., 2008. Int J Obes., 33(7): 758-67). In this sense, the use of microorganisms from the lactic acid bacteria and bifidobacteria group to treat obesity or the associated diseases has been proposed in different publications or patents. International patent application WO2007/043933 proposes that the *Lactobacillus* F19 and NCFB 1748 and *B. lactis* Bb12 strains can be used to control body weight and reduce appetite in the form of fermented milks; nevertheless, milk proteins and the calcium contained therein could be those responsible for the effect rather than the strains and the effects are only based on the modification of the expression of genes related to metabolism in the small intestine; however, obesity involves many other organs and tissues. Patent application US 20100061967 A1 also proposes the use of bacteria which modulate the expression of peptides regulating satiety only in the gastrointestinal tract. Another patent application (W02009153662) proposes the use of bifidobacteria and lactobacilli in diabetes exclusively based on the capacity of these microorganisms to reduce peripheral tissue inflammation without taking the effects at the level of the central nervous system or other processes involved in the origin and evolution of this pathology into account. Patent application US20100150890 proposes the use of probiotic bacteria to stimulate the function of the sympathetic nervous system, such that metabolism and energy expenditure are accelerated; nevertheless, the sympathetic tone is also increased in some obese patients. Patent application US20100111915 proposes the generic use of probiotic bacteria in childhood to prevent obesity based on their bifidogenic effect (increasing in the total amount of bifidobacteria in the intestine), without providing any fundament as to the manner in which a simple general increase of bifidobacteria can modify specific processes which lead to the development of obesity; moreover, if it is taken into account that the properties of bifidobacteria are specific to each strain. Furthermore, studies conducted relating to the aforementioned patent document have not been conducted with a *Bifidobacterium* genus strain but with *Lactobacillus* genus strains or their combination with *B. lactis* Bb12 and modifications to the diet, so the results cannot be attributed to the bifidobacteria (US20100111915; Luoto et al., 2010. Br J Nutr., 103 (12) : 1792-9; Luoto et al. 2010. Int J Obes (Lond). Mar 16) and, in any case, they are strains different from those of the present invention. Another patent application US20050112112 proposes the use of microorganisms generating polymers from mono- and disaccharides, reducing the particular absorption of these compounds by the individual either by using substances such as chitosan combined with bifidobacteria to specifically reduce cholesterol absorption (JP10306028), which are all partial ways to reduce the problem of obesity associated with specific components of the diet.

Therefore, there is still a problem of finding more suitable strategies to prevent and/or treat diseases such as overweight, obesity, and associated pathologies, such as diabetes, dyslipidemia, hepatic steatosis and metabolic syndrome for example, acting together on the relation thereof to the poor working of the immune system and the connection thereof with glucose metabolism and insulin resistance at the peripheral and central level, and with alterations in the accumulation of lipids in blood and peripheral tissues which are responsible for different chronic pathologies.

### Summary of the Invention

The present invention relates to the *Bifidobacterium pseudocatenulatum* CECT 7765 strain, to the cell components, metabolites, secreted molecules of said strain, and to the combinations thereof with other microorganisms, and to the compositions comprising the aforementioned products, as well as to its use for the prevention and/or treatment of overweight and/or obesity and associated alterations such as diabetes, dyslipidemia, hepatic steatosis, metabolic syndrome or immune system dysfunction with effects on other pathologies such as infections. The present invention also relates to the use of said strain for the prevention and/or treatment of these alterations when they are not associated with a problem of overweight and/or obesity.

The CECT 7765 strain, which belongs to the *B. pseudocatenulatum* species, has immunological properties comparatively more favorable than other strains of the same species, of other *Bifidobacterium* genus species and of other lactic bacteria genera. The CECT 7765 strain significantly induces a lower production of TNF-alpha proinflammatory cytokine in macrophages compared with strains of other species and of the same species (between approximately 2.5 and 12.6 times lower; Table 1); furthermore, it also induces a lower production of MCP1 (between approximately 1.2 and 38.2 times lower; Table 1). As discussed in the state of the art section, the synthesis of these cytokines and chemokines by macrophages has been directly related for example with obesity, diabetes, dyslipidemia and other related metabolic disorders. In addition, the CECT 7765 strain induces the synthesis of anti-inflammatory cytokines, such as IL10, inversely related with these pathologies in a higher proportion than other *Bifidobacterium* genus strains (between 1.9 and 4.8 times higher; Table 2). The immunological properties of the selected bacterium are not common to all bacterial strains of the *Lactobacillus* and *Bifidobacterium* genera, and they make it particularly suitable for application in the treatment and prevention of overweight and/or obesity and of associated pathologies, when they are presented together with or independently of the obesity and overweight, because the common feature of all of them is their association with a condition of low-grade chronic inflammation.

Unlike the state of the art, the present invention approaches the treatment of obesity with a multifactorial perspective and, furthermore, acts on new key targets for the prevention and/or treatment of this pathology or associated pathologies not described for any known *Bifidobacterium* genus strain. The most interesting fact is that none of the known strains of this genus or species has proven to be useful for the simultaneous and effective treatment of all the conditions indicated throughout the present invention.

Therefore, the present invention provides to the state of the art a high-value *B. pseudocatenulatum* species strain for the treatment of overweight and/or obesity as well as certain associated pathologies such as, for example but not being limited to, diabetes, hepatic steatosis, dyslipidemia or metabolic syndrome.

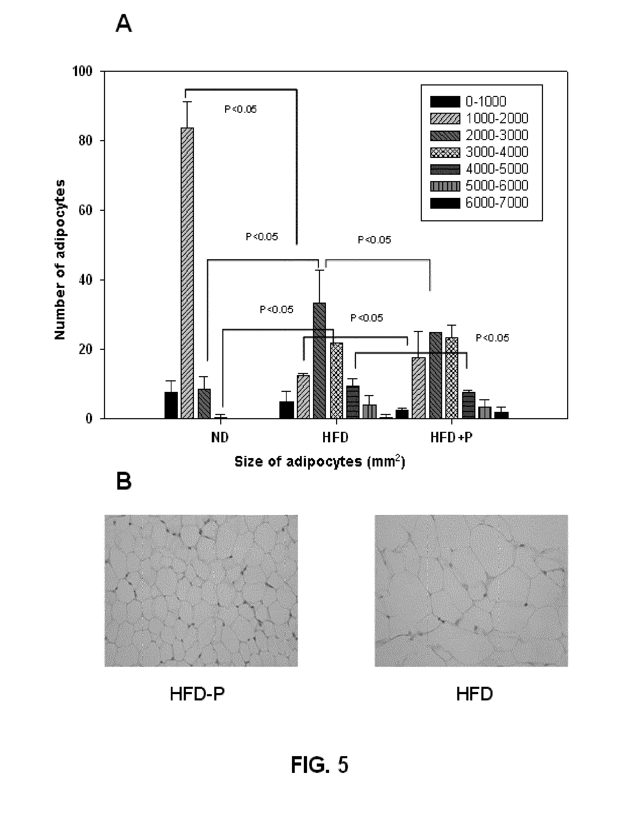

Essentially, the advantages presented by using the *B*. *pseudocatenulatum* CECT 7765 strain of the present invention are the following:
- The administration of the *B. pseudocatenulatum* CECT 7765 strain reduces the size of the adipocytes in obese and non-obese subjects (see Example 4). In particular, the administration of the CECT 7765 strain to obese animals leads to an increase in small-sized adipocytes (1000-2000 µm²), whereas in obese animals that have not been administered the strain there is an increase in large-sized adipocytes (4000-6000 µm²) (Example 4, Figure 5).

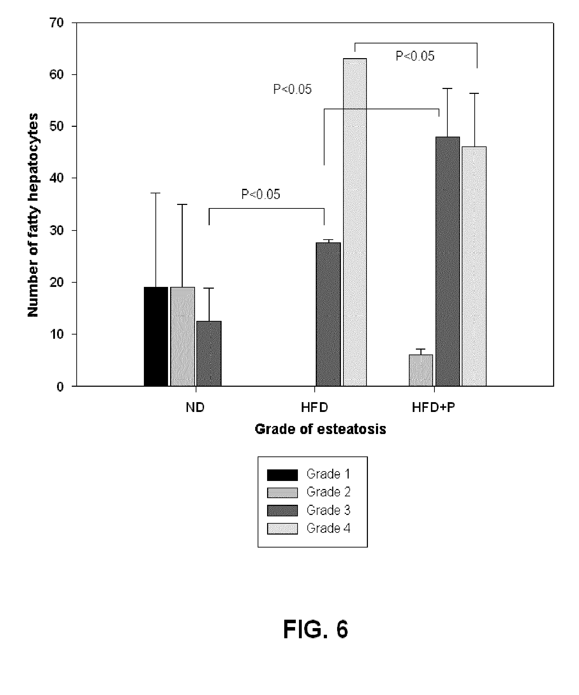

The fact that the CECT 7765 strain reduces the size of the adipocytes demonstrates that it is useful for the treatment of adipocyte hypertrophy which, if maintained over time and if it occurs in a large number of adipocytes, can cause overweight and obesity. This is because large-sized adipocytes secrete a higher concentration of growth factors triggering adipogenesis through preadipocyte differentiation, generating a feedback process. Hypertrophic adipocytes furthermore produce an abnormally high concentration of inflammatory cytokines and chemokines (TNF-α, MCP-1, IL-6, resistin, etc.) which inhibit insulin signaling in hepatocytes and cause insulin resistance and other complications. The increased size of adipocytes is also related with the increased supply of fatty acids to the liver, which leads to hepatic steatosis and its complications. Therefore, the strain can likewise contribute to preventing or improving these associated pathologies. - The administration of the strain object of the invention leads to a reduction of the fat accumulated in the liver in obese and non-obese subjects (Example 4, Figure 6). Specifically, the strain reduces the number of grade 4 maximum fat content hepatocytes (> 66%) and an increase of grade 3 hepatocytes (34-66% fat content); however, in obese animals that have not been administered the strain, the proportion of the type of hepatocytes is the inverse, the maximum fat content hepatocytes predominating. In control animals, the administration of the strain increases grade 2 hepatocytes (fat content < 33%) and reduces grade 3 hepatocytes (34-66% fat content), which is the inverse of what occurs in animals that have not received the strain. It is thus demonstrated that the administration of the strain reduces the total accumulation of fat in the liver induced or not induced by diet.

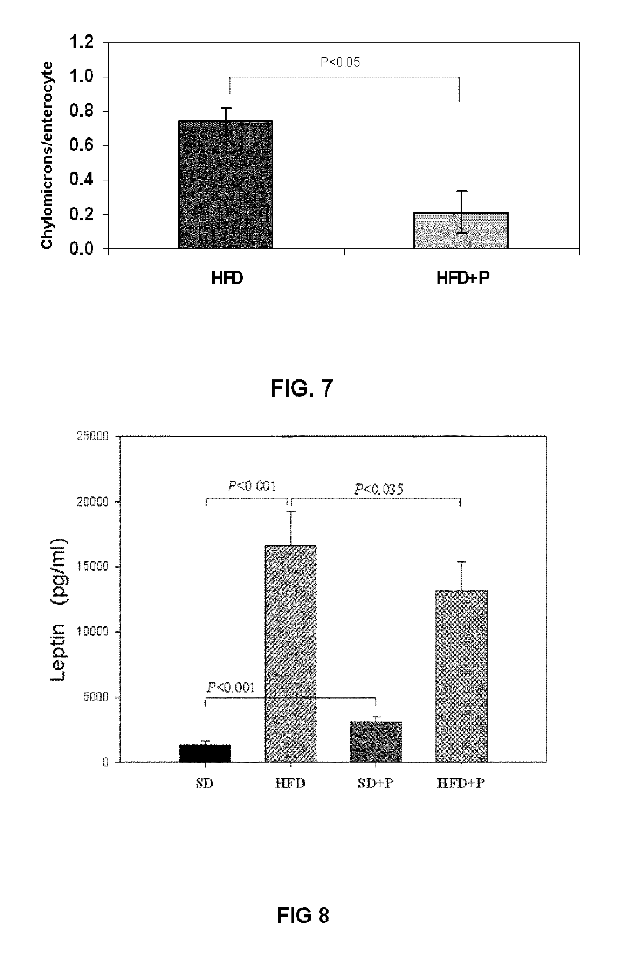

Therefore, the CECT 7765 strain can be used for the prevention and/or treatment of hepatic steatosis. This pathology can be considered a pathology associated with diets with a high energy load and with obesity (is present in 60-90% of obese subjects), but there are cases in which it is not caused by overweight and/or obesity, but, for a non-limiting example, can be caused by infections and nutritional or hereditary metabolic disorders.
- The *B. pseudocatenulatum* CECT 7765 strain reduces the number of chylomicrons in enterocytes, i.e., it reduces the amount of fat in the diet that can be absorbed and passed to lymph and blood in the form of chylomicrons and, thus, to peripheral tissues (Example 6, Figure 7). This property is also reflected in a reduction in the concentrations of peripheral blood triglycerides (Example 5). In addition to being able to be a cause of overweight and/or obesity as it causes an increase in the accumulation of fat in adipose tissue, the increased absorption of fat from the diet can be the cause of other pathologies without causing overweight or obesity, such as for example, and without limiting the scope of the invention, atherosclerosis, dyslipidemia, metabolic syndrome, cardiovascular pathologies and other disorders deriving from the relation between lipid metabolism and glucose metabolism. Dyslipidemia can also be a consequence of not only fat absorbed from the diet but of other metabolic disorders, such as adipocyte insulin resistance which, without necessarily being associated with obesity, makes the adipocytes release fatty acids that will be used in the liver to increase triglyceride synthesis and secretion and increase of peripheral blood triglycerides. Dyslipidemia can also present in subjects that are genetically predisposed to this metabolic disorder, without necessarily being associated with obesity, insulin resistance, or the increase in the absorption of fat from the diet. Therefore, the CECT 7765 strain can be effective in the prevention and/or treatment of diseases related with the excessive absorption of fats from the diet and for the prevention and/or treatment of dyslipidemia (e.g. hypertriglyceridemia and hypercholesterolemia).
- The CECT 7765 strain regulates glucose metabolism disorders and increases the peripheral blood glucose concentration (hyperglycemia) related with insulin synthesis and function (Example 5). The increase in glucose can occur, among other causes, because of insulin resistance or lack of insulin synthesis but it is not necessarily associated with obesity.
- The CECT 7765 strain improves the working of innate and adaptive immune system cells, increasing their response capacity to infectious agents, antigens or allergens in obese and non-obese subjects. In particular, the administration of the strain to animal models of obesity induced by a high-fat diet improves, among others, the function of macrophages in phagocytosis and in cytokine synthesis (Example 3, Figures 2 and 3). The strain object of the invention also improves the function of adaptive immune system dendritic cells and T-cells (Example 3, Figure 4).

Therefore, the CECT 7765 strain has an additional positive effect because it can be useful for the prevention and treatment of infections and the improvement of protective responses for example in vaccination and immunization processes, because these functions of the immune system are altered in overweight and obese subjects. Furthermore, the strain of the invention can be useful for the treatment or prevention of other diseases (for example, diabetes) presenting with immunosuppression (fundamentally of macrophages, dendritic cells and T-cells) that is associated or not associated with obesity and overweight.
- The CECT 7765 strain is capable of inducing a lower amount of proinflammatory proteins at the peripheral and central level in obese and normal weight subjects treated with said strain with respect to those not treated with the strain. Therefore, the strain object of the invention also reduces the TNF-α synthesis in the peripheral system and in the central nervous system, the synthesis of which is increased in obesity and other pathologies and contributes to the development of insulin and leptin resistance, inhibiting their anorexigenic effects (reducing the feeling of hunger) and their function in regulating body weight and glucose metabolism (Example 3). The strain also reduces the peripheral blood leptin concentration in obese subjects in whom the concentration is increased, and it favors inflammation and increases the concentration in normal weight subjects in whom it inversely contributes to reducing appetite and consumption and to increasing energy expenditure and lipid oxidation and, therefore, to reducing body weight (Example 3, Figure 8).

Therefore, the CECT 7765 strain regulates the production of proteins and hormones (cytokines, chemokines and adipokines), the synthesis of which is altered in obesity and in certain diseases associated with it such as, for non-limiting examples, diabetes, dyslipidemia, metabolic syndrome, cardiovascular diseases and steatosis, both in peripheral blood and in the central nervous system, and in other diseases not necessarily associated with overweight and/or obesity, and it can therefore be used for the treatment and prevention of these pathologies.
- Furthermore, the CECT 7765 strain restores the composition of the intestinal microbiota by normalizing alterations associated with overweight and obesity and the inflammatory effect caused by these alterations and has been related with weight gain, insulin resistance, metabolic endotoxemia, hepatic steatosis and the alteration of the intestinal barrier function (Example 3). The strain of the invention can be also used to reducing overgrowth of pathogenic or opportunistic intestinal enterobacteria which can primarily or secondarily be associated with other underlying pathologies or which are a risk for the triggering thereof. Therefore, the CECT 7765 strain can additionally be used in the prevention and treatment of infections and diseases associated with alterations in the intestinal microbiota.

One aspect of the present invention relates to a *B*. *pseudocatenulatum* strain with accession number CECT 7765. Said strain was deposited in the *Colección Española de Cultivos Tipo* (Spanish Type Culture Collection) (CECT) on 21 July 2010 and it was granted accession number CECT 7765. The address of said international depositing authority is: Universidad de Valencia / Edificio de Investigación / Campus de Burjassot / 46100 Burjassot (Valencia).

The scientific classification of the CECT 7765 strain of the present invention is: Kingdom: *Bacterium* / Phylum: *Actinobacteria* / Order: *Bifidobacteriales* / Family: *Bifidobacteriaceae* / Genus: *Bifidobacterium* / Species: *pseudocatenulatum.*

The features of said strain are:
- The substrates which the CECT 7765 bacterium oxidizes or ferments are: D-arabinose, ribose, B-methyl-D-xyloside, galactose, D-glucose, α-methyl-D-mannoside, N-acetyl glucosamine, amygdalin, arbutin, esculin, cellobiose, maltose, lactose, melibiose, melezitose and xylitol.
- The CECT 7765 strain has the following enzymatic activities: ortho nitrophenyl-ßD-galactopyranosidase and arginine dihydrolase.
- The strain grows in a temperature range comprised between 31 and 42°C, with optimal growth at 37°C.
- The strain grows in a pH range comprised between 5 and 8, with optimal growth at pH 7.

Furthermore, the strain is stable in gastrointestinal stress conditions (acid pH and high bile concentration). Its viability after incubation in gastric conditions (pepsin 3g/l at pH 3 and 2.5) for mean gastric emptying time (2 h) is 64-95% and its growth in the presence of bile salts (0.5 and 1%) is maintained between 80 and 90%. It is also resistant to technological conservation process conditions (freezing, lyophilization, etc.), and to food preparation conditions (refrigeration, lyophilization, fermentation, etc.) and it grows in different industrial-scale microorganism production media. For example, the strain shows growth in milk virtually equal to that obtained in MRS commercial laboratory medium and a greater logarithmic unit in GEM industrial production medium. *In vivo* it is capable of surviving the intestinal transit after oral administration, showing reductions of only 1-2 logarithmic units in relation to the initial dose administered, depending on the subjects and on the type of sample analyzed and time elapsed after administration. All these properties assure its viability, persistence and effectiveness in the intestine.

Another preferred embodiment of the present invention relates to a derivative strain of the *B. pseudocatenulatum* CECT 7765 strain, where said strain maintains or improves the capacities described throughout the present invention. The derivative microorganism can be produced naturally or intentionally by mutagenesis methods known in the state of the art, such as, for example but not being limited to, growing the original microorganism in the presence of mutagenic or stress-causing agents, or by means of genetic engineering aimed at modifying specific genes. According to a more preferred embodiment, the derivative strain of the *B. pseudocatenulatum* CECT 7765 strain is a genetically modified mutant. The terms mutant strain or derivative strain can be used indifferently.

The *B. pseudocatenulatum* CECT 7765 strain or any mutant or derivative thereof can be used in any form which exercises the described effects, such as, for example, according to a preferred embodiment of the present invention, the *B*. *pseudocatenulatum* CECT 7765 strain is in the form of (culturable or non-culturable) viable cells, or according to another preferred embodiment of the invention, the strain is in the form of non-viable cells ("dread" cells inactivated by any technique known in the state of the art, such as, for example but not being limited to, heat, freezing or ultraviolet radiation).

Hereinafter reference can be made to any of the bacterial strains of the *B. pseudocatenulatum* species described in the preceding preferred embodiments and aspect as the "strain of the present invention" or the "strain of the invention".

Another aspect of the present invention relates to the microorganism combination comprising the strain of the invention. The microorganism combination is a set of cells of the strain of the invention, or at least one cell of the strain of the invention, together with a set of cells of another strain of the same species or of different species or another taxonomical group of microorganisms. The cells of the microorganism combination can be non-viable or viable and be in any phase of the state of development or growth (latent, exponential, stationary, etc.), regardless of their morphology.

A preferred embodiment of the present invention relates to the microorganism combination where said combination comprises at least another microorganism other than the strain of the invention, for example but not being limited to, the microorganism which can be part of said combination is:
- at least another *Bifidobacterium* genus strain, for a non-limiting example, the *B. longum* CECT 7347 strain or other *B*. *pseudocatenulatum, B. catenulatum, B. breve, B. longum* subsp. *longum, B. longum* subsp. *infantis, B. lactis* subsp. *lactis, B. lactis* subsp. *animalis,* or *B. adolescentes* species strains.
- at least one lactic bacterium of intestinal, food or environmental origin. The lactic bacterium is selected from the list comprising, but not limited to, a bacterium of the *Lactobacillus, Lactococcus, Enterococcus, Propionibacterium, Leuconostoc, Weissella, Pediococcus or Streptococcus* genus.
- at least one strain of other phylogenetic groups, genera or species of prokaryotes of intestinal, food or environmental origin, such as for example but not being limited to *Archaea, Firmicutes, Bacteroidetes, Proteobacteria, Actinobacteria, Verrucomicrobia, Fusobacteria, Methanobacteria, Spirochaetes, Fibrobacteres, Deferribacteres, Deinococcus, Thermus, Cyanobacteria, Methanobrevibacterium, Peptostreptococcus, Ruminococcus, Coprococcus, Subdoligranulum, Dorea, Bulleidia, Anaerofustis, Gemella, Roseburia, Catenibacterium, Dialister, Anaerotruncus, Staphylococcus, Micrococcus, Propionibacterium, Enterobacteriaceae, Faecalibacterium, Bacteroides, Parabacteroides, Prevotella, Eubacterium, Akkermansia, Bacillus, Butyrivibrio or Clostridium;*
- at least one fungus or yeast strain, such as for example but not being limited to, one belonging to the *Saccharomyces, Candida, Pichia, Debaryomyces, Torulopsis, Aspergillus, Rhizopus, Mucor or Penicillium* genus.

Hereinafter reference can be made to any of the microorganism combinations described in the preceding paragraph as the microorganism combination of the present invention" or the "microorganism combination of the invention".

Another aspect of the present invention relates to the cell components, metabolites, secreted molecules or any combinations thereof, obtained from the strain of the invention, or from the microorganism combination of the invention. The present invention also contemplates the combination of the cell components, metabolites, secreted molecules or any combinations thereof, obtained from the CECT 7765 strain, or from the microorganism combination of the invention with other food, plant product and drug components.

The cell components of the bacterium could include the cell wall components (such as, for example but not being limited to, peptidoglycan), the nucleic acids, membrane components, or others such as proteins, lipids and carbohydrates and combinations thereof, such as lipoproteins, glycolipids or glycoproteins. The metabolites include any molecule produced or modified by the bacterium as a consequence of its metabolic activity during growth, use in technological processes (for example but not being limited to food or drug preparation processes), during product storage or during gastrointestinal transit. Examples of these metabolites are, but are not limited to, organic and inorganic acids, proteins, peptides, amino acids, enzymes, lipids, carbohydrates, lipoproteins, glycolipids, glycoproteins, vitamins, salts, metals or nucleic acids. The secreted molecules include any molecule exported or released out by the bacterium during growth, its use in technological processes (for example for food or drug preparation), product storage or gastrointestinal transit. Examples of these molecules include, but are not limited to, organic and inorganic acids, proteins, peptides, amino acids, enzymes, lipids, carbohydrates, lipoproteins, glycolipids, glycoproteins, vitamins, salts, metals or nucleic acids.

Another aspect of the present invention relates to a composition comprising the strain of the invention or the microorganism combination of the invention or the cell components, metabolites, secreted molecules of the strain of the invention or any combinations thereof.

The composition, generally defined, is a set of components which consists of at least the strain of the invention at any concentration or of at least the cell components, metabolites, secreted molecules of the strain of the invention or any combinations thereof.

The pharmaceutical composition is a set of components which consists of at least the strain of the invention at any concentration or of at least the cell components, metabolites, secreted molecules of the strain of the invention or any combinations thereof, having at least one application in improving the physical or psychological well-being of a subject, which involves a improvement of his/her general state of health.

The term medicinal product has a more limited meaning that the meaning of "pharmaceutical composition", as defined in the present invention, because the medicinal product necessarily involves a preventive or therapeutic effect, i.e., a physiological effect, on the subject. The term "medicinal product" will be duly defined below.

Another preferred embodiment of the present invention relates to the composition, where said composition is a pharmaceutical composition. In an even more preferred embodiment, the pharmaceutical composition further comprises at least one pharmaceutically acceptable carrier and/or excipient.

The term "excipient" refers to a substance which aids in the absorption of any of the components of the composition of the present invention, stabilizes said components or aids in the preparation of the pharmaceutical composition in the sense of giving it consistency or providing flavors making it more palatable. Therefore, the excipients could have the function of keeping the components bound to one another, such as for example starches, sugars or celluloses, a sweetening function, a colorant function, the function of protecting the medicinal product, such as for example to isolate it from the air and/or moisture, the function of a filler for a tablet, capsule or any other presentation form, such as for example dibasic calcium phosphate, a disintegrating function to facilitate the dissolution of the components and their intestinal absorption, without excluding another type of excipients not mentioned in this paragraph. Therefore, the term "excipient" is defined as that material which, included in galenic forms, is added to active ingredients or to their associations to enable their preparation and stability, to modify their organoleptic properties or to determine the physicochemical properties of the pharmaceutical composition and its bioavailability. The "pharmaceutically acceptable" excipient must allow the activity of the compounds of the pharmaceutical composition, i.e., it must be compatible with said components.

The "galenic form or dosage form" is the presentation to which the active ingredients and excipients are adapted to constitute a medicinal product. It is defined by the combination of the form in which the pharmaceutical composition is presented by the manufacturer and the form in which it is administered.

The "carrier" or vehicle, is preferably an inert substance. The function of the carrier is to facilitate the incorporation of other compounds, to allow better dosage and administration or to give the pharmaceutical composition consistency and shape. Therefore, the carrier is a substance which is used in the medicinal product to dilute any of the components of the pharmaceutical composition of the present invention to a specific volume or weight; or which is capable, even without diluting said components, of allowing a better dosage and administration or of giving the medicinal product consistency and shape. When the presentation form is liquid, the pharmaceutically acceptable carrier is the diluent.

Furthermore, the excipient and the carrier must be pharmacologically acceptable, i.e., the excipient and the carrier are allowed and evaluated such that they do not harm the organisms to which they are administered.

In another even more preferred embodiment, the pharmaceutical composition further comprises another active substance. In addition to the requirement of therapeutic efficacy, where said pharmaceutical composition may need to use other therapeutic agents, there may be additional fundamental reasons which make it greatly necessary or advisable to use a combination of a compound of the invention and another therapeutic agent. The term "active ingredient" is any material, regardless of whether it is of human, animal, plant, or chemical origin or of another type, attributed with suitable activity for constituting a medicinal product.

In each case the presentation form of the medicinal product will be adapted to the type of administration used, so the composition of the present invention can be presented in the form of solutions or any other clinically allowed dosage form and in a therapeutically effective amount. The pharmaceutical composition of the invention can be formulated in solid, semisolid, liquid or gaseous forms, such as tablet, capsule, powder, granule, ointment, solution, suppository, injection, inhalant, gel, microsphere or aerosol. According to an even more preferred embodiment of the present invention, the pharmaceutical composition is presented in a form suitable for oral administration.

The form suitable for oral administration refers to a physical condition which can allow oral administration. Said form suitable for oral administration is selected from the list comprising, but not limited to, drops, syrup, herbal tea, elixir, suspension, extemporaneous suspension, drinkable vial, tablet, capsule, granulate, cachet, pill, pellet, pastille, troche or lyophilisate.

Another possibility is for the pharmaceutical composition to be presented in a form suitable for sublingual, nasal, intrathecal, bronchial, lymphatic, rectal, transdermal or inhaled administration. The strain of the invention, the microorganism combination of the invention, the cell components, metabolites, secreted molecules or any combinations thereof, obtained from the strain of the invention, or from the microorganism combination of the invention, can be associated, for example but not being limited, with liposomes or micelles.

In the sense used in this description, the expression "therapeutically effective amount" refers to that amount of the component of the pharmaceutical composition which, when administered to a mammal, preferably a human, is sufficient to cause the prevention and/or treatment, as defined below, of a disease or pathological condition of interest in the mammal, preferably a human. The therapeutically effective amount will vary, for example, according to the activity of the strain of the invention, of the microorganism combination of the invention, of the cell components, metabolites, secreted molecules or any combinations thereof, in any presentation form, the therapeutically effective amount will also vary according to the metabolic stability and duration of the action of the compound, age, body weight, general state of health, gender and diet of the patient, the method and time of administration, the excretion rate, the combination of drugs, the severity of the particular disorder or pathological condition, and the subject undergoing therapy, but it can be determined by a person skilled in the art according to his/her own knowledge and this description.

Another preferred embodiment of the present invention relates to the composition, where said composition is a nutritive composition. A more preferred embodiment of the present invention relates to the nutritive composition where said composition is a food, a nutraceutical, a supplement, a probiotic or a symbiotic.

The term "nutritive composition" of the present invention refers to that food which, regardless of the nutrients provided to the subject taking it, beneficially affects one or several functions of the organism, such that it provides a better state of health and well-being. Consequently, said nutritive composition can be intended for the prevention and/or treatment of a disease or of disease-causing factor. Therefore, the term "nutritive composition" of the present invention can be used as a synonym of functional food or food for specific nutritional purposes or medicinal food.

As it is used herein, the term "nutraceutical" refers to isolated substances of a food and used in a dosed manner having a beneficial effect on health.

As it is used herein, the term "probiotic" refers to live microorganisms which, when supplied in suitable amounts, are beneficial for the health of the host organism.

As it is used herein, the term "symbiotic" refers to those foods containing a mixture of prebiotic and probiotic. They generally contain a prebiotic component favoring growth and/or metabolic activity and in summary the effect of the probiotic combined therewith, such as, for a non-limiting example, the association of the fructooligosaccharides or galactooligosaccharides with bifidobacteria.

The term "supplement", a synonym of any of the terms "dietary supplement", "nutritional supplements" or "food supplement", is a "food ingredient" intended to complement the diet. Some examples of dietary supplements include but are not limited to vitamins, minerals, botanical products, amino acids and food components such as enzymes and glandular extracts. They are not presented as replacements of a conventional food or as a single component of a meal or of the diet but as a complement of the diet.

According to an even more preferred embodiment, the food is selected from the list comprising: dairy product, plant product, meat product, a snack, chocolate, beverage or baby food. The dairy product is selected from the list comprising, but not limited to, fermented milk product (for example but not limited to yogurt or cheese) or non-fermented milk product (for example but not limited to ice-cream, butter, margarine, whey). The plant product is, for example but not being limited to, a fermented or non-fermented cereal in any presentation form. The beverage can include but is not limited to any fruit juice or non-fermented milk.

Another more preferred embodiment of the present invention relates to any of the compositions described in the invention, where said composition has a concentration of the strain of between 10³ and 10¹⁴ colony forming units (cfu) per gram or milliliter of final composition. The concentration of the strain is that concentration which is therapeutically effective or nutritionally effective, as appropriate. The nutritive composition and the pharmaceutical composition can be formulated in, but are not limited to, solid, semisolid, liquid or gaseous forms, such as a tablet, capsule, microcapsule, powder, granule, ointment, solution, paste, suppository, injection, inhalant, gel, microsphere or aerosol.

Hereinafter, reference can be made to any of the compositions, the general composition, pharmaceutical composition or nutritive composition, defined in preceding paragraphs by means of the term "composition of the present invention" or "composition of the invention".

Another aspect of the present invention relates to the use of a CECT 7765 strain of *B. pseudocatenulatum* species for the production of a pharmaceutical composition, of a medicinal product or of a nutritive composition. Another aspect of the present invention relates to the use of the strain of the invention, or of the microorganism combination of the invention, or of the cell components, metabolites, secreted molecules, or any combinations thereof, or of the composition of the invention, for the production of a nutritive composition, of a medicinal product or of a nutritive composition. Any pharmaceutical composition defined in the preceding paragraphs can be used for the production of a medicinal product.

The medicinal product to which the present invention relates can be for human or veterinary use. The "medicinal product for human use" is any substance or combination of substances which has properties for the treatment or prevention of diseases in human beings or which can be used in human beings or be administered to human beings for the purpose of restoring, correcting or modifying physiological functions by exerting a pharmacological, immunological or metabolic action, or of establishing a medical diagnosis. The "medicinal product for veterinary use" is any substance or combination of substances which has curative or preventive properties with respect to animal diseases or which can be administered to the animal for the purpose of restoring, correcting or modifying its physiological functions by exerting a pharmacological, immunological or metabolic action, or of establishing a veterinary diagnosis. The "pre-mixtures for medicated feed" prepared for being incorporated in feed will also be considered "veterinary medicinal products".

Another preferred embodiment of the present invention relates to the use of a *B. pseudocatenulatum* species strain, of the strain of the invention CECT 7765, or of the microorganism combination of the invention, or of the cell components, metabolites, secreted molecules, or any combinations thereof, or of the composition of the invention for the production of a medicinal product or for the production of a nutritive composition, for the prevention and/or treatment of overweight, obesity or for the prevention and/or treatment of any pathology or dysfunction (for example immune system dysfunction) associated therewith.

As it is understood herein, the term "treatment" refers to controlling the effects caused as a consequence of a disease or pathological condition of interest in a subject (preferably mammal, and more preferably a human) which includes:
(i) inhibiting the disease or pathological condition, i.e., stopping its development;
(ii) alleviating the disease or the pathological condition, i.e., causing the regression of the disease or the pathological condition or its symptomatology;
(iii) stabilizing the disease or the pathological condition.

As it is understood herein, the term "prevention" consists of preventing the onset of the disease, i.e., preventing the disease or the pathological condition from occurring in a subject (preferably mammal, and more preferably a human), particularly when said subject has a predisposition for the pathological condition.

As it is used herein, the term "overweight" refers to a pathology characterized in that the subject has a body mass index (BMI) equal to or greater than 25. The BMI is a measure of association between the weight and height of an individual. The BMI has the following formula for its calculation: Mass (Kg) / height² (m). Overweight is characterized by a BMI between ≥ 25 and < 30.

When the BMI is equal to or greater than 30, the subject suffers from "obesity". Obesity is classified in different levels, considering that subjects with BMI > 40 suffer from morbid obesity. Obesity is a clinical condition in which the energy reserves stored in the adipose tissue of humans and other mammals exceed healthy limits. Lipid accumulation leads to fat deposition in different tissues, overweight, obesity and to a series of pathologies associated with said overweight or obesity, such as, for example but not being limited to, type 2 diabetes mellitus and gestational diabetes, dyslipidemia (preferably hyperlipidemia and hypercholesterolemia), cardiovascular disease, hypertension, fatty liver (preferably non-alcoholic fatty liver or hepatic steatosis, non-alcoholic steatohepatitis, cirrhosis or hepatitis), metabolic syndrome, cancer, infections, etc. The relationship between said pathologies and their association with overweight or with obesity is well known in the state of the art (such as for example in WO/2010086454 or WO/2008119110). Therefore, the use of the medicinal product, the pharmaceutical composition or the nutritive composition in the prevention and/or treatment of pathologies associated with overweight and/or obesity is justified because said association has been widely demonstrated and, therefore, the use of a *B*. *pseudocatenulatum* species strain or strain of the invention could prevent the onset of diseases the cause of which is overweight and/or obesity, as a person skilled in the art would expect.

Although BMI is commonly used to determine whether or not a subject suffers from obesity, there are other parameters for that purpose. The absolute waist circumference (the subject suffers from obesity when it is >102 cm in men [central obesity] and >88 cm in women) or the waist-hip ratio (the subject suffers from obesity when it is >0.9 for men and >0.85 for women) are used as measures of central obesity. An alternative way for determining obesity is to measure the percentage of body fat (the subject suffers from obesity when he/she has approximately >25% body fat in a man and approximately >30% of body fat in a woman). Central obesity (masculine type or waist obesity predominantly, characterized by a high waist-hip radius) is an important risk factor for metabolic syndrome, which is a series of alterations and risk factors which strongly predispose a subject, but does not limit one, to suffering cardiovascular disease and type 2 diabetes mellitus.

The effects of obesity on health are considered the result of an increase of the fat mass in different cells and tissues and are furthermore the result of a chronic inflammation condition and immune system dysfunction which, together with metabolism disorders, are the cause of different pathologies mentioned above.

Another preferred embodiment of the present invention relates to the use of a *B*. *pseudocatenulatum* species strain or of the strain of the invention, or of the microorganism combination of the invention, or of the cell components, metabolites, secreted molecules, or any combinations thereof; or of the composition of the invention, for the production of a medicinal product or for the production of a nutritive composition, to reduce the growth and differentiation of adipose tissue in obese or overweight subjects, and a stage prior to overweight or obesity, and it is therefore used for the prevention and/or treatment of adipocyte hypertrophy. As demonstrated in Example 4 and in Figure 5, the strain object of the invention reduces the size of the adipocytes, the increase (hypertrophy) of which in certain stages of life (especially during childhood and adolescence) especially favors the development of overweight and obesity in adult age and other associated complications. In particular, the administration of the CECT 7765 strain to obese animals leads to an increase in the number of small-sized adipocytes, and to a reduction of the number of large-sized adipocytes (Example 4, Figure 5).

Another preferred embodiment of the present invention relates to the use of a *B*. *pseudocatenulatum* species strain or of the strain of the invention, or of the microorganism combination of the invention, or of the cell components, metabolites, secreted molecules, or any combinations thereof, or of the composition of the invention, for the production of a medicinal product or for the production of a nutritive composition, for the treatment of hepatic steatosis or fatty liver. As demonstrated in Example 4 of the present invention, the administration of the *B. pseudocatenulatum* CECT 7765 strain both to animal models of obesity and to control animals (non-obese) reduces the number of hepatocytes with high fat accumulation. Overall, this means that the strain of the invention reduces fat accumulation in the liver.

A *B. pseudocatenulatum* species strain or the strain of the invention can be used for the treatment or prevention of hepatic steatosis or fatty liver, defined in a preceding paragraph as a disease associated with overweight and/or obesity. The present invention also relates to the prevention and/or treatment of pathologies related with the worsening of hepatic steatosis, such as, for example but not being limited to, non-alcoholic hepatitis, steatohepatitis, fibrosis, cirrhosis, terminal liver disease or liver carcinoma. Furthermore, a *B. pseudocatenulatum* species strain or the strain of the invention can be used for these or other pathologies presenting with lipid accumulation in the liver and inflammation, but which do not necessarily present in obese or overweight subjects, but are a consequence of other disorders. Such disorders include, for example, but are not limited to, nutritional disorders (for example but not being limited to malabsorption, protein-calorie malnutrition or parenteral nutrition), hereditary or non-hereditary metabolic disorders (for example but not being limited to type 2 diabetes mellitus, abetalipoproteinemia, or systemic carnitine deficiency), diseases caused by the exposure to drugs (for example but not being limited to corticoids or ibuprofen) or toxins (for example but not being limited to alcohol), chronic or acute hepatitis due to infections, cirrhosis, fibrosis, terminal liver disease, liver carcinoma, or pituitary gland disorders. In particular, steatosis affects approximately 50% of patients with type 2 diabetes mellitus.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition for the prevention and/or treatment of a disease caused by alterations in blood lipid concentrations (for example dyslipidemia) and, preferably, blood triglyceride concentrations, with respect to a control, therefore it is used to normalize the blood concentration thereof. The medicinal product or nutritive composition is preferably used for the treatment of dyslipidemia (synonymous with dyslipidemia). Dyslipidemia is preferably hypertriglyceridemia or hypercholesterolemia. Dyslipidemia is a pathological condition the only common element of which is a lipid metabolism disorder, with its subsequent alteration in blood lipid and lipoprotein concentrations. Dyslipidemia may or may not be associated with obesity and with the consumption of high-fat diets and with the increase of fat absorption. In turn, these alterations are related with a higher risk of cardiovascular diseases and diabetes, among other pathologies. The strain of the invention both in normal subjects and in obese subjects reduces lipid absorption and blood triglycerides levels, proving to be effective for the described applications, as is demonstrated in Example 5.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition, to reduce the amount of lipids absorbed from the diet, with respect to an untreated control.

As shown in Example 6, the strain of the invention reduces the number of chylomicrons in the intestinal enterocytes, i.e., it reduces the amount of fat from the diet which is absorbed by more than 50% (Example 6; Figure 7). Chylomicrons are the form in which the lipids from the diet are packaged and transported from the intestine to the lymph and to the blood to be used by the peripheral tissues and, through this mechanism, the administered strain would limit their absorption and accumulation in the organism. The absorption of the fat in the diet, in addition to being able to be the cause of overweight and/or obesity by causing an increase in its accumulation in adipose tissue, can be the cause of other pathologies without causing obesity, such as for example and without limiting the scope of the invention: atherosclerosis, which is characterized by a thickening of the intima of an artery with plaques where the fat builds up, and dyslipidemia, characterized by alterations in plasma lipid concentrations (triglycerides and/or cholesterol and associated lipoproteins), pathologies associated with a higher cardiovascular risk, or other alterations derived from the relationship of lipid metabolism with glucose metabolism (for example but not being limited to insulin resistance or diabetes).

A preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition for the prevention and/or treatment of a disease caused by higher blood glucose levels with respect to a control, therefore it is used to reduce blood glucose concentration (hyperglycemia) with respect to an untreated subject and to maintain its normal physiological levels and to regulate postprandial glycemic response.

The increase in fasting blood glucose (hyperglycemia) and the alteration of the postprandial glycemic response can be caused by insulin resistance (subjects who produce sufficient insulin but the body does not respond normally) or by a lack of insulin synthesis, with or without obesity, due to other metabolic disorders or interactions with drugs. Example 5 of the present invention provides experimental support to this preferred embodiment. The term "disease caused by higher blood glucose levels" relates to a health alteration caused by higher blood glucose concentrations than what should be expected of a healthy individual with normal glucose values, i.e., approximately between 72 and 110 mg/dl blood or 4 - 7 mmol/l while fasting, or approximately < 180 mg/dl (or 10 mmol/l) if measured an hour and a half after meals. Said values are approximate mean values because the variation experienced by the conditions characteristic of each subject must be taken into account. The disease caused by higher blood glucose levels is selected from the list comprising, but not limited to, neuropathy (damage to the nerves in the extremities and/or organs), retinopathy (damage to the retina in the eyes), nephropathy (damage to the kidney which can cause kidney failure), cardiovascular diseases (for example hypertension or myocardial infarction), cerebrovascular disease (for example, cerebral thrombosis).

Another preferred embodiment of the present invention relates to the use of a *B*. *pseudocatenulatum* species strain or of the strain of the invention, or of the microorganism combination of the invention, or of the cell components, metabolites, secreted molecules, or any combinations thereof, or of the composition of the invention, for the production of a medicinal product or for the production of a nutritive composition, for the prevention and/or treatment specific for diabetes. A more preferred embodiment relates to the prevention and/or treatment of type 2 diabetes mellitus, a pathology associated with overweight and/or obesity, though not necessarily.

Type 2 diabetes mellitus is characterized by the relative deficiency in insulin production and insulin sensitivity in the tissues and, therefore, a deficient peripheral use of glucose. Type 2 diabetes mellitus represents 80%-90% of all diabetic patients. It often develops in adult stages in life and is very commonly associated with obesity. Several drugs and other causes can, however, cause this type of diabetes. For example, diabetes is frequently associated with the prolonged administration of corticoids, frequently associated with untreated hemochromatosis, and gestational diabetes not always associated with obesity.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition, to reduce growth and differentiation of adipose tissue in obese or overweight subjects, and a stage prior to overweight or obesity , and it is therefore used for the prevention and/or treatment of metabolic syndrome. Metabolic syndrome refers to the set of metabolic disorders which together increase the risk of diabetes and cardiovascular disease, in including the combination of obesity, dyslipidemia (e.g. triglyceridemia and hypercholesterolemia) and hyperglycemia. As is demonstrated in preceding examples, the strain of the invention is useful for the prevention and simultaneous treatment of these disorders and, therefore, of metabolic syndrome.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition for the prevention and/or treatment of a disease associated with an alteration of the innate and adaptive immune response, with respect to that of control subjects, therefore it is used to improve the function of the innate and adaptive immune system with respect to an untreated subject. This pathology is preferably overweight, obesity and the associated disorders leading to an alteration of these immune functions. Example 3 shows experimental data in this respect.

The term "disease associated with the reduction of the innate and adaptive immune response" refers to diseases presenting with immunosuppression of the function of the innate and adaptive immune system.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition, for the prevention and/or treatment of a disease associated with a higher production of proinflammatory proteins, with respect to a control. Examples 2 and 3 show experimental data in this respect.

Examples of proinflammatory proteins include but are not limited to cytokines, chemokines and adipokines. The proinflammatory proteins are preferably selected from the list comprising IL-1, IL-6, IL-8, IL-12, IL-16, TNF-alpha or MCP1 and leptin, or any combinations thereof. The proinflammatory proteins are more preferably selected from the list comprising TNF-alpha, IL-6, MCP1 and leptin or any combinations thereof. The term "disease associated with a higher production of proinflammatory proteins" refers to diseases which are caused by at least the production of a protein involved in the inflammation (proinflammatory) of different types of tissues. Some of the diseases associated with a higher production of proinflammatory proteins are also associated with overweight and/or obesity, such as, for example but not being limited to, type-2 diabetes, gestational diabetes, metabolic syndrome, fatty liver, non-alcoholic hepatitis, hypertension, dyslipidemia, cardiovascular diseases, atherosclerosis, steatohepatitis, or cancer. Other associated diseases with a higher production of proinflammatory proteins are not associated with overweight and/or obesity or can present in the absence of obesity such as, for example, but not being limited to, the aforementioned diseases (for example, diabetes) and other diseases such as allergic inflammation.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition, to reduce the concentration of leptin in an obese subject and/or to increase the concentration of said hormone in a non-obese subject, with respect to an untreated control. In the non-obese subject, the increase in leptin leads to a reduction in consumption and to the increase in the energy expenditure and lipid oxidation. In the obese subject, in contrast, the reduction in the concentration of leptin contributes to normalizing metabolic disorders and reducing inflammation.

Another preferred embodiment of the present invention relates to the use of a *B*. *pseudocatenulatum* species strain, or of the strain of the invention, or of the microorganism combination of the invention, or of the cell components, metabolites, secreted molecules, or any combinations thereof, or of the composition of the invention, for the production of a medicinal product or for the production of a nutritive composition, to restore the composition of the intestinal microbiota, or to reduce the concentration of enterobacteria in the intestinal content. In an even more preferred embodiment, the restoration of the composition of the intestinal microbiota, or the reduction of the concentration of enterobacteria in the intestinal content, is carried out in an overweight subject, obese subject or in a subject with any pathology associated therewith.

The restoration of the intestinal microbiota can be based, for a non-limiting example, on the reduction of the concentration of enterobacteria in the intestinal content as well as on the increase in bifidobacteria and/or lactobacilli, with respect to an untreated control. As can be observed in Example 3.6, the administration of the strain of the invention leads to an increase in the concentration of total bifidobacteria of at least 1 logarithmic unit in the colon and to a reduction of the concentration of potentially inflammatory bacteria such as enterobacteria of at least 0.5 logarithmic units. This also entails a reduction of the proinflammatory signals that can be transmitted from the intestine to peripheral tissues (for example the liver) which can be affected in obese or non-obese subjects by different pathologies.

Throughout the description and the claims the word "comprises" and its variants do not seek to exclude other technical features, additives, components or steps. For persons skilled in the art, other objects, advantages and features of the invention will be inferred in part from the description and in part from the practice of the invention. The following drawings and examples are provided by way of illustration and do not seek to limit the present invention.

### Description of the Drawings

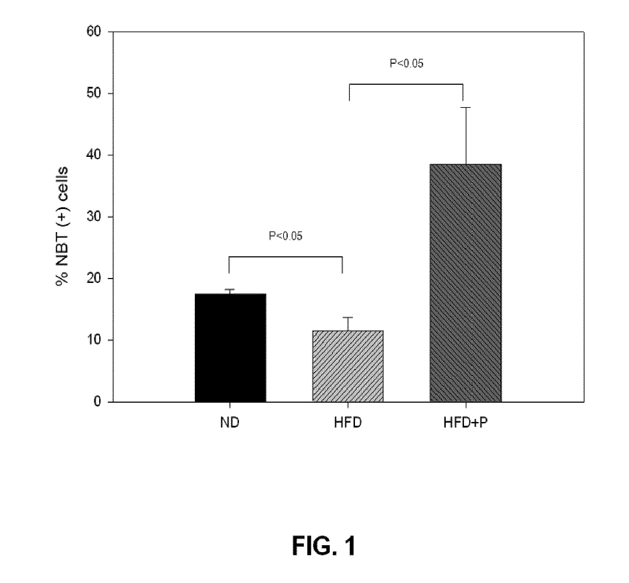

Figure 1 shows the effect of the administration of the *B*. *pseudocatenulatum* CECT 7765 strain (10⁸ cfu/day) in obese C57BL/6 mice (n=6/group) for 7 weeks on the respiratory burst of macrophages involved in phagocytosis.
   ND, control animals with standard diet; HFD, high-fat diet; HFD+P, high-fat diet + *B. pseudocatenulatum* CECT 7765.

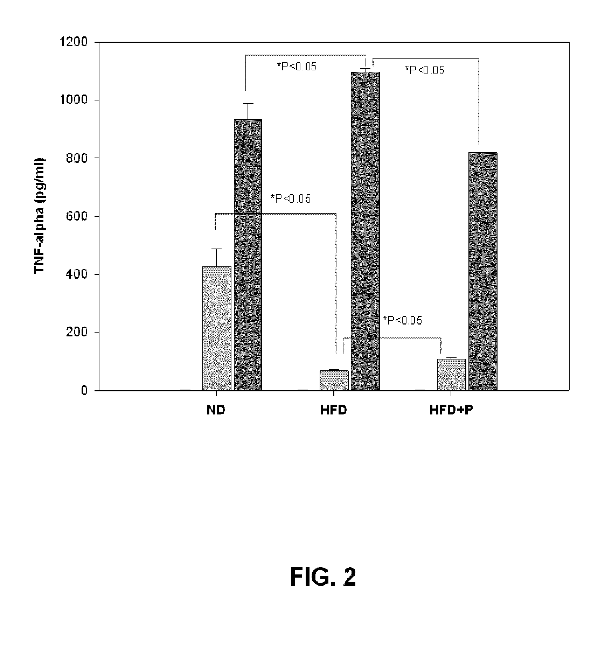

Figure 2 shows the effect of the administration of the *B*. *pseudocatenulatum* CECT 7765 strain (10⁸ cfu/day) in obese C57BL/6 mice (n=6/group) for 7 weeks on the function of macrophages in cytokine synthesis (TNF-alpha) with respect to different stimuli (lipopolysaccharide [LPS] and feces).
   ND, control animals with standard diet; HFD, high-fat diet; HFD+P, high-fat diet + *B. pseudocatenulatum* CECT 7765. Black bars: control; light grey: LPS; dark grey: feces.

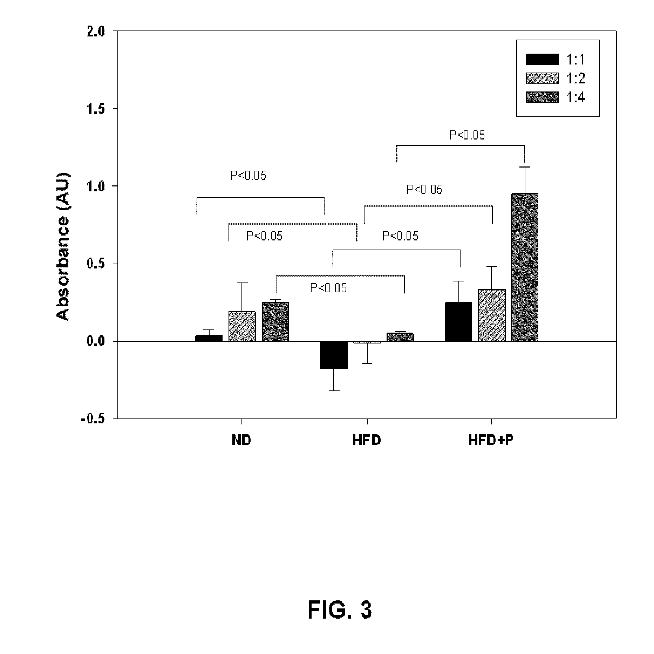

Figure 3 shows the effect of the administration of the *B*. *pseudocatenulatum* CECT 7765 strain (10⁸ cfu/day) in obese C57BL/6 mice (n=6/group) for 7 weeks on the interaction of dendritic cells with T-cells and their proliferation capacity.
   CD4+ T-cells (L) were incubated with mature dendritic cells (DC) of different experimental groups of obese C57BL/6 mice (n=6/group) that were administered the strain (10⁸ cfu/day) for 7 weeks. The cell ratio (L/DC) in the mixture was 1:1, 1:2 and 1:4.
   ND, control animals with standard diet; HFD, high-fat diet; HFD+P, high-fat diet + *B. pseudocatenulatum* CECT 7765.

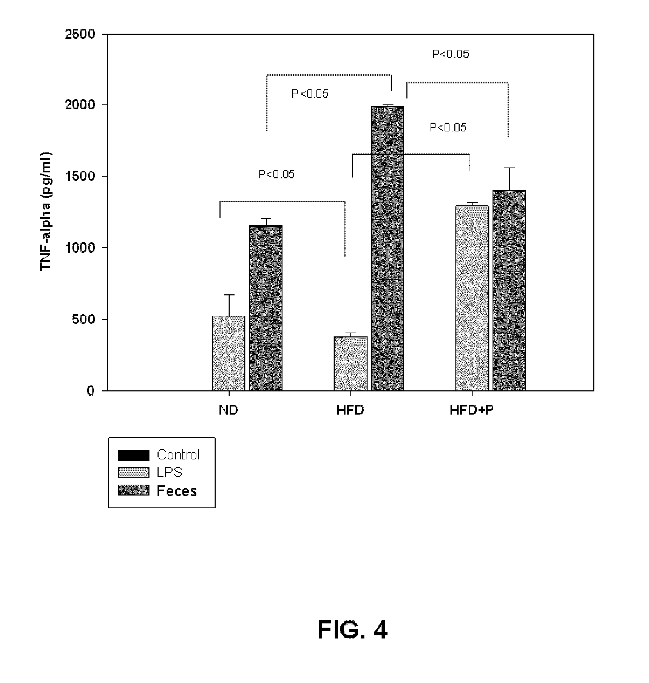

Figure 4 shows the effect of the administration of the *B*. *pseudocatenulatum* CECT 7765 strain (10⁸ cfu/day) in obese C57BL/6 mice (n=6/group) for 7 weeks on the function of dendritic cells in cytokine synthesis with respect to different stimuli (LPS and feces).
   ND, control animals with standard diet; HFD, high-fat diet; HFD+P, high-fat diet + *B. pseudocatenulatum* CECT 7765.
Figure 5 shows the effect of the administration of the *B*. *pseudocatenulatum* CECT 7765 strain (10⁸ cfu/day) in obese C57BL/6 mice (n=6/group) for 7 weeks on the development of adipocytes, classified by size ranges.
   ND, control animals with standard diet; HFD, high-fat diet; HFD+P, high-fat diet + *B. pseudocatenulatum* CECT 7765.
Figure 6 shows the effect of the administration of the *B*. *pseudocatenulatum* CECT 7765 strain (10⁸ cfu/day) in obese C57BL/6 mice (n=6/group) for 7 weeks on the development of steatosis (lipid accumulation in the liver).
   The number of fatty hepatocytes according to the degree of fat accumulation in the cell is shown in a histological section of liver tissue stained with hematoxylin eosin. ND, control animals with standard diet; HFD, high-fat diet; HFD+P, high-fat diet + *B. pseudocatenulatum* CECT 7765.
Figure 7 shows the effect of the administration of the *B*. *pseudocatenulatum* CECT 7765 strain (10⁸ cfu/day) in obese C57BL/6 mice (n=6/group) for 7 weeks on the number of chylomicrons formed in the enterocytes in histological sections stained with hematoxylin eosin.
   HFD, animals with high-fat diet; HFD+P, high-fat diet + *B*. *pseudocatenulatum* CECT 7765.
Figure 8 shows the effect of the administration of the *B*. *pseudocatenulatum* CECT 7765 strain (10⁸ cfu/day) in obese C57BL/6 mice (n=6/group) and control mice for 7 weeks on the peripheral blood leptin concentration.
   L, Leptin expressed in pg/ml; HFD, animals with high-fat diet; HFD+P, high-fat diet + *B. pseudocatenulatum* CECT 7765; SD, animals with standard diet; SD+P animals with standard diet + *B*. *pseudocatenulatum* CECT 7765.

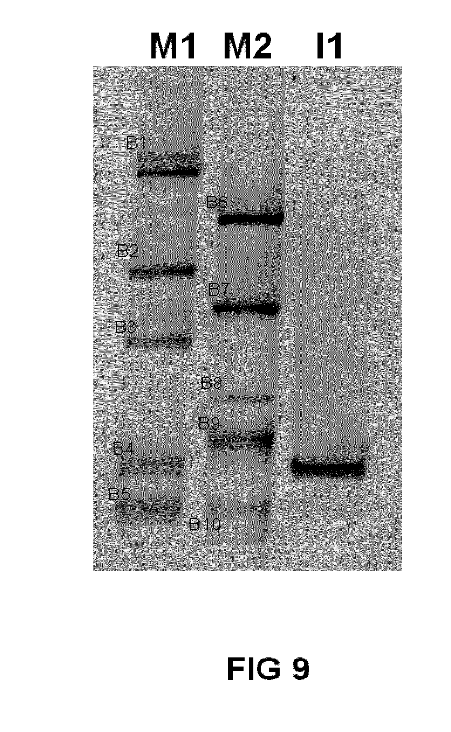

Figure 9 shows the identification of the species of the strain of the invention by electrophoresis in denaturing gradient gel electrophoresis (DGGE).
   Column M1 shows different bands Bx corresponding with DNA fragments of the reference microorganisms indicated below: B1 is *B. adolescentis* LMG 11037T; B2 is *B. angulatum* LMG 11039T; B3: *B. longum* subsp *infantis* CECT4551T; B4: *B. pseudocatenulatum* CECT 5776; B5: *B. animalis* subsp. *lactis* DSM 10140T.
   In column M2, B6 is *B. bifidum* LMG 11041T; B7 is *B. longum* subsp. *longum* CECT 4503T; B8 is *B. catenulatum* LMG 11043T; B9 is *B. dentium* CECT 687; B10 is *B. animalis* subsp. *animalis* LMG 10508T.
   In column I1, the band shows the strain of the invention CECT 7765

### Examples

The invention will be illustrated below by means of assays performed by the inventors. The following specific examples provided in this patent document serve to illustrate the nature of the present invention. These examples are included only for illustrative purposes and must not be interpreted as limitations to the invention herein claimed. Therefore, the described examples do not seek to limit the field of application thereof.

### Example 1. Isolation and identification of the B. pseudocatenulatum CECT 7765 strain

*Bifidobacterium* genus strains were isolated from the feces of healthy breastfeeding mice that had not consumed foods containing bifidobacteria for at least the month prior to the analysis and that had not been subjected to treatments with antibiotics. The samples were kept at 4°C and were analyzed in less than two hours after they were collected. Two grams of each of them were diluted in 10 mM phosphate buffer containing a concentration of 130 mM NaCl (PBS) and they were homogenized in a Lab-Blender 400 stomacher (Seward Medical, London, UK), for 3 minutes and were diluted in peptone water. 0.1 ml aliquots of different decimal dilutions were inoculated in MRS agar (Man Rogose and Sharpe; Scharlau, Barcelona) containing 0.05% of cysteine (Sigma, St. Louis, MO; MRS-C), and 80 µg/ml of mupirocin. After 48 h of incubation at 37°C in anaerobic conditions (AnaeroGen, Oxoid, UK) isolated colonies were selected and their identity was confirmed by studying their morphology under Gram staining. The identity of the isolates was confirmed by sequencing the 16S RNA gene from total DNA. The fragment sequenced was amplified using the primers 27f (5' AGAGTTTGATCCTGGCTCAG-3': SEQ ID NO: 2) and 1401r (5'-CGGTGTGTACAAGACCC-3': SEQ ID NO: 3) and it was purified using the GFX™PCR commercial system (Amershan, Bioscience, UK). Primers 530f (5'-GTGCCAGCAGCCGCGG-3': SEQ ID NO: 4) and U-968f (5'-AACGCGAAGAACCTTAC-3': SEQ ID NO: 5) were further used for sequencing according to the methods described by other authors (Gerhard et al., 2001. Appl. Environ. Microbiol., 67: 504-513; Satokari et al., 2001. Appl. Environ. Microbiol. 67, 504-513; Favier et al., 2002. Appl. Environ. Microbiol., 68: 219-22). Sequencing was performed using an ABI 3700 automatic DNA sequencer *(Applied Biosystem, Foster City, CA).*

The 1.28 kb sequence of the 16S ribosomal RNA gene of the CECT 7765 strain is SEQ ID NO: 1. The search for more closely related sequences was conducted in the GenBank database using the BLAST algorithm (Altschul et al., 1990. J. Mol Biol., 215: 403-410).

According to the comparison of SEQ ID NO: 1 with respect to the most similar sequences, an identity of 99% was obtained with respect to other bacteria of the *B. pseudocatenulatum* species (for example with respect to the *B. pseudocatenulatum* B1279 strain (GenBank accession number NR_037117.1). These results indicate that the strain of the present invention may very likely belong to said species.

The identification of the species was also confirmed by DGGE as described in a prior study (Satokari et al., 2001. Applied and Environmental Microbiology, 67, 504-513). The sequence of the 16S rRNA gene (520 pbb) was amplified with the primers Bif164 and Bif662-GC and the amplified fragments were separated in Universal Mutation Detection System electrophoresis equipment (Bio-Rad, Richmond, CA). A 45-55% denaturing gradient was established in the gel in which 100% corresponded with a 7 M concentration of urea and 40% formamide (vol/vol). The electrophoretic mobility was compared with collection strains used as reference for each species.

As shown in Figure 9, the electrophoretic mobility of the band of the strain of the invention (gel lane I1: *B*. *pseudocatenulatum* CECT 7765) coincides with that of another strain of the same species used as reference (*B. pseudocatenulatum* CECT 5776).

The strain of the invention was molecularly typed by means of RAPD analysis using primers M13 (5'-GAGGGTGGCGGTTCT-3': SEQ ID NO: 6) and Del (5'-CCGCAGCCAA-3': SEQ ID NO: 7) and according to the methodology described above (Hoffmann et al., 1998. Zentralbl Bakteriol. 288, 351-60; Svec et al. 2010. Antonie Van Leeuwenhoek. 98: 85-92). The profiles of the randomly amplified DNA fragments demonstrated that the strain object of the invention (*B. pseudocatenulatum* CECT 7765) is different from other strains of the same species.

### Example 2. Selection of the B. pseudocatenulatum CECT 7765 strain according to its capacity for modulating the response of macrophages involved in low-grade chronic inflammation in vitro 2.1. Preparation of intestinal bacteria cultures and supernatants

The strains were inoculated in 10 ml of MRS broth (*Scharlau Chemie* S.A., Barcelona, Spain) containing 0.05% of 1% cysteine (MRS-C) with a 24 h culture and they were incubated for 22 h at 37°C in anaerobic conditions. (*AnaeroGen; Oxoid, Basingstoke, UK*). The cells were collected by centrifugation (6,000 g, 15 minutes), were washed two times in PBS (10 mM sodium phosphate, 130 mM sodium chloride, pH 7.4), and were resuspended in PBS containing 20% glycerol. Aliquots of these suspensions were frozen with liquid nitrogen and they were conserved at -80°C. The number of viable cells after the freezing-thawing cycle was determined by counting in MRSC agar plates after incubated for 48 h. Viability was greater than 90% in all cases. Each aliquot was used for a single assay. For the purpose of evaluating the effects of dead bacteria, some of the aliquots were cold-inactivated (3 -20°C freezing and thawing cycles) and heat-inactivated (30 minutes at 80°C). The pH values of the supernatants obtained were adjusted to 7.2 with NaOH and were sterilized by filtration (0.22-µm pore size, Millipore, Bedford, MA) to remove the possible presence of viable cells. Aliquots of the cell-free supernatants were conserved at -80°C until use.

### 2.2. Macrophage culture and stimulation.

Cells from the Raw 264.7 murine macrophage cell line were grown in Dulbecco's modified Eagle medium (DMEM, Sigma, USA), supplemented with 10% fetal bovine serum (Gibco, Barcelona, Spain), streptomycin (100 µg/ml, Sigma) and penicillin (100 U/ml, Sigma). To conduct the stimulation experiments, the cells were incubated at a concentration of 10⁶ cells/ml in 24-well flat bottom polystyrene plates (Corning, Madrid, Spain) at 37° C, at 5% CO₂. Suspensions of living and dead bacteria of 1 x 10⁶ colony forming units (cfu)/ml and supernatant volumes of 150 µl were used as stimulus. Lipopolysaccharide (LPS) purified from *E. coli* 0111:B4 (Sigma, St. Louis, MO) at a concentration of 1 µg/ml was used as positive control. The cytokine production in non-stimulated PBMCs was assayed as negative control. Each type of stimuli was assayed in duplicate in each experiment. The culture supernatants were collected by centrifugation, fractioned and stored in aliquots at -20°C until cytokine and chemokine detection.

### 2.3. Cytokine and chemokine determination

The concentrations of cytokines (TNF-α, IL-6, IL10 and MCP1) of the supernatants were measured by means of ELISA kits (BD Biosciences, San Diego, CA) according to the manufacturer's instructions.

The strain object of the invention was selected among others from the *Lactobacillus* and *Bifidobacterium* genera due to the capacity thereof for inducing low concentrations of proinflammatory molecules (TNF-α and IL-6) and chemotactic molecules (MCP1) involved in the migration of macrophages to adipose tissue and in the chronic inflammation condition associated with obesity which causes resistance to the action of insulin and leptin (Table 1). The CECT 7765 strain was the one which induced the production of a lower concentration of TNF-α, IL-6 and it was one of the two strains which induced lower production of MCP1 (Table 1). The strain of the invention was also selected because it induces the synthesis of high concentrations of anti-inflammatory and regulatory cytokines (IL-10, Table 2) by macrophages, which can contribute to reducing inflammation in the context of obesity (Table 2). The immunological properties of the selected bacterium are not common to all the intestinal bacteria of the *Lactobacillus* and *Bifidobacterium* genera, or to those of other strains of the same species and, therefore, they make it particularly suitable for application thereof in the treatment and prevention of overweight, obesity and associated alterations, as well as other diseases as discussed in preceding sections.

The increase in the production of TNF-α, IL-6 and MCP1 has been associated with overweight, obesity and related pathologies, but not only is it associated with said pathologies but also with any pathology caused by an increase in TNF-α, IL-6 and MCP1 with respect to a control, as described in preceding paragraphs of the description.

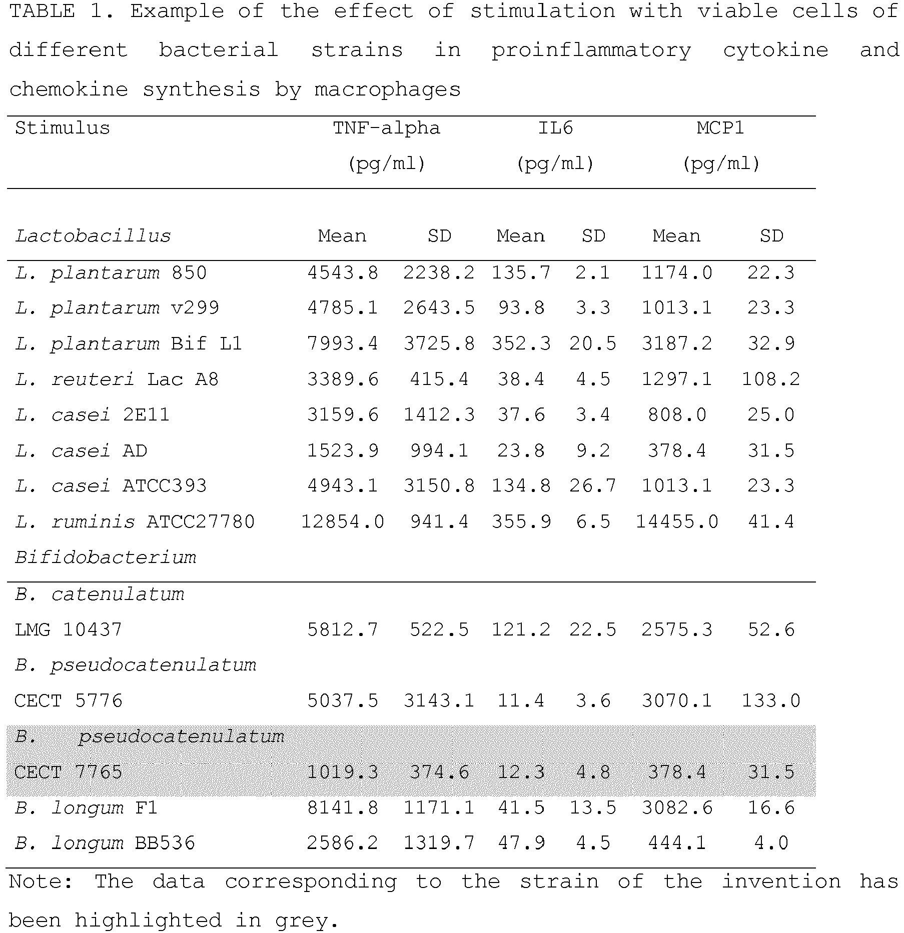

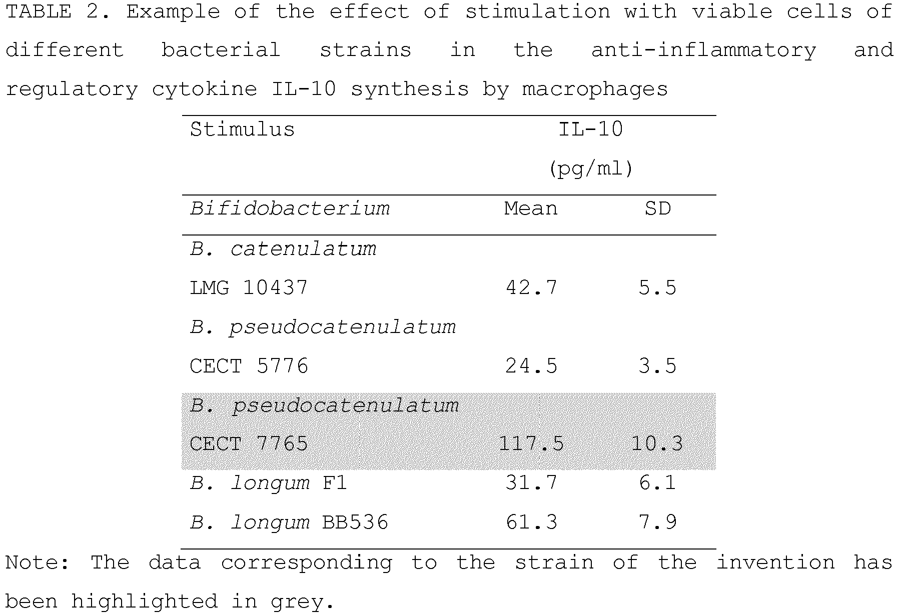

### Example 3. Effect of the administration of B. pseudocatenulatum CECT 7765 strain on the function of immune system cells, on immunological and endocrine parameters in peripheral blood and in the central nervous system, and on the composition of intestinal microbiota and the inflammatory properties thereof

### 3.1. Preparation of cultures of the strain object of the invention

The CECT 7765 strain was grown in MRS broth (*Scharlab,* SL-Barcelona, Spain) supplemented with 0.05% (w/v) cysteine at 37°C in anaerobic conditions (*AnaeroGen; Oxoid, Basingstoke, UK*) for 22 h. The cells were collected by centrifugation (6,000 g for 15 minutes), were washed with phosphate buffer solution (PBS, 10 mM sodium phosphate, 130 mM sodium chloride, pH 7.4), and were resuspended in 10% skim milk. Aliquots of these suspensions were frozen with liquid nitrogen and were conserved at -80°C until use. The viability of the bacteria was checked by counting in MRS agar plates with 0.05% cysteine after 48 hours of incubation and it was approximately 90%. Each aliquot was thawed only once.

### 3.2. Animal model of obesity and sampling

C57BL-6 adult male mice (6-8 weeks; Harlan Laboratories) were used. The animals were kept at controlled temperature (23°C) with a 12-h light/dark cycle and in an atmosphere of 40-50% relative humidity. The C57BLACK6 mice, abbreviated as C57BL-6 or black 6, is the most widely used endogamic strain of laboratory mice for being genetically manipulated in the study of human diseases.

The groups of obese animals were generated by means of feeding with a high-fat diet (HFD) which provided 60% of the energy in the form of lipids (60/Fat, Harlan Laboratories) for 7 weeks, whereas the non-obese animals were administered a conventional diet. The mice had free access to water and to the food. The weight was monitored weekly. The experiments were conducted according to the animal ethics committee standards.

The animals were randomly divided into 4 groups (n=6/group): fed with a conventional diet (controls), controls that were administered the strain object of the invention (controls-strain), fed with a high-fat diet (obese) and obese that were administered the CECT 7765 strain (obese-strain). The strain was administered at a daily dose of 10⁸ cfu/day by means of stomach tube for 7 weeks. The control and obese groups were administered water in the same way as placebo.

After this time, the animals were anesthetized and sacrificed by cervical dislocation and different biological samples were taken. The immunological parameters (cytokines, adipokines and chemokines) in peripheral blood were determined by means of using ELISA. The concentration of inflammatory cytokines (TNF-alpha) was also determined in the supernatant of brain samples previously homogenized with a polytron by means of an ELISA assay, as described in the preceding section. Fecal samples were also taken to determine the effect of the administration of the strain on the microbiota composition and it immunological response-stimulating effect on *in vitro* cultures of macrophages, dendritic cells and T-cells obtained as described below.

### 3.3. Evaluation of the effect on macrophages

For the purpose of demonstrating the effect of the administration of the CECT 7765 strain on the improvement of the response of innate immune system cells, macrophages were obtained by aseptically injecting Dulbeco's Modified Eagles Medium solution (DMEM) (SigmaTM- St. Louis, MO/USA), by intraperitoneal route, supplemented with 10% fetal bovine serum inactivated at 56°C for 30 minutes (Gibco, Barcelona, Spain), 100 µg/ml of streptomycin and 100 U/ml of penicillin (*Sigma Chemical Co.*). The macrophages obtained from each experimental group of mice were adjusted to a concentration of 1x10⁵ cells/ml in medium DMEM and after incubation for 1 h at 37°C in 5% CO₂ atmosphere, the wells were washed with serum-free DMEM to remove the non-adhered cells. The adhered cells were incubated for 24 h and, after this period, were stimulated with feces (dilution 1/9 in PBS) of each experimental group of mice and with 1 µg/ml of *Salmonella typhimurium* LPS (Sigma Chemical Co, Madrid, Spain) as positive control. In parallel, non-stimulated macrophages were evaluated for the purpose of knowing basal cytokine production. After stimulation, the supernatants were collected and the concentrations of the following cytokines were determined therein: TNF-α, IL6 and IL-10 by ELISA (Ready SET Go! Kit, BD Bioscience, San Diego, CA, USA).

### 3.4. Evaluation of the effect on dendritic cells and T-cells

For the purpose of demonstrating the effect of the administration of the strain object of the invention on the improvement of the capacity of dendritic cells to stimulate the T-cell response and, therefore, the adaptive immune response, the capacity of mature dendritic cells to induce the proliferative response of CD4+ T-cells in a mixed lymphocyte reaction was determined. The assay was conducted by comparing the responses of the cells extracted from obese and control mice that were or were not administered the strain object of the invention as described above.

The dendritic cells were generated from bone marrow of tibias and femurs of the mice. The tibias and femurs of each mouse were extracted and the surrounding tissue was aseptically removed. After cutting the ends, the bone marrow was extracted flushing it with PBS, using a syringe and a needle 0.45 mm in diameter. The cells obtained were washed once with PBS and aliquots of 10⁶ cells diluted in RPMI, supplemented with antibiotics (penicillin 100 IU/ml and streptomycin 100 µg/ml), 10% FBS and 20 ng/ml of mouse GM-CSF, were seeded in 100 mm flasks. On the third day, 10 ml of culture medium were added and on the seventh day, the medium was replaced with fresh medium. On the eighth day, the non-adhered cells were harvested by means of gentle pipetting. The cells were washed with PBS and resuspended in culture medium without mouse GM-CSF.

The dendritic cells (DC) were activated by adding LPS (100 ng/ml) during 24 h before performing the mixed lymphocyte reaction. Mature DC were used to stimulate CD4+ T-cells. The CD4+ T-cells were isolated from the spleens of C57BL/6 mice 7-8 weeks of age. After being extirpated, the spleens were suspended in PBS with FBS and passed through a nylon mesh, the cell suspension obtained was washed once and resuspended in a lysis buffer for 5 minutes. After two washings with PBS, the CD4+ T-cells (CT) were immunogenetically separated by positive selection with L3T4-CD4+ microbeads (Miltenyi Biotec GmbH, Bergisch Gladbach, Germany) according to the manufacturer's instructions.

To perform the mixed lymphocyte reaction, aliquots of DC were distributed in 96-well plates in triplicate to stimulate, in each case, 1x10⁵ CD4+ T-cells (L) in the following ratios (L/DC): 1:1, 1:2, 1:4 in 100 µl of culture medium, and they were incubated at 37°C for 72 h in 5% CO₂ atmosphere. DC and CD4+ T-cells with and without ConA (5 µg/ml; Sigma), used as a mitogen, were used as controls. Lymphocyte proliferation was determined with an ELISA kit (BrdU-colorimetric assay; Roche, Diagnostic, Germany) and was quantified by measuring absorbance at 440 nm.

The strain of the invention improves the working of innate and adaptive immune system cells when administered *in vivo* to subjects with obesity induced by the diet, increasing their capacity to respond to infectious agents, antigens or allergens. In particular, the administration of the strain to animal models of obesity induced by a high-fat diet improves, among others, the function of the macrophages in phagocytosis and in cytokine synthesis (Figure 1 and 2). The administration of the strain increases the respiratory burst of peritoneal macrophages in response to a foreign allergen or stimulus (yeast/pathogen), improving the phagocytic capacity and therefore immunological defenses (Figure 1). This capacity is significantly reduced in obese animals with respect to the non-obese controls (Figure 1). Earlier studies also show that the respiratory burst of phagocytic cells responsible for removing pathogens is also altered in subjects with diabetes (Marhoffer et al., 1992. Diabetes Care, 15(2): 256-60). Furthermore, the culture of peritoneal macrophages extracted from obese and control animals and their stimulation in *vitro* with the lipopolysaccharide (LPS) of a pathogen shows that the administration of the strain object of the invention improves cytokine, such as TNF-α, synthesis responsible for stopping a possible infection (Figure 2). The strain object of the invention also improves the function of dendritic cells and T-cells when administered *in vivo.* The dendritic cells extracted from obese mice that were administered the strain, incubated in the presence of T-cells in different ratios (1:1, 1:2 and 1:4), increase their proliferation and activation capacity, properties which are reduced in obese animals that were not administered the strain (Figure 3). The best working of the dendritic cells in the obese animals that were administered the strain is also demonstrated because after their stimulation with LPS *in vitro* they are capable of inducing higher secretion of cytokines (for example TNF-α) involved in the response to pathogens (Figure 4). These properties of the strain object of the patent make it suitable because the functionality of dendritic cells and T-cells is altered in obesity and associated diseases such as diabetes. In particular, the dendritic cells have functional alterations associated with weight gain, characterized by a reduction of their capacity to present antigens and to stimulate allogeneic T-cells (Macia et al., 2006. J Immunol., 177(9): 5997-6006; Verwaerde et al., 2006. Scand J Immunol., 64(5): 457-66). The proinflammatory properties of naive T-cells against a stimulus (mitogen or antigen) are increased, being able to contribute to the low-grade chronic inflammatory state associated with obesity, and, in contrast, the T-cells previously exposed to antigens have a proliferation defect and preferably secrete type Th2 cytokines. All this explains the high incidence of infections in obese subjects and the lack of memory T-cell-mediated response to vaccination and infections (Karlsson et al., 2010. J Immunol., 184: 3127-33). The function of T-cells is also deficient in diabetics, showing reduced capacity to proliferate in response to a stimulus and to synthesize IL2 (Chang and Shaio. 1995. Diabetes Res Clin Pract., 28(2): 137-46).

The strain of the invention administered *in vivo* regulates cytokine, chemokine and adipokine production, the synthesis of which is altered in obesity and associated diseases in peripheral blood. The changes induced in animal models of obesity and controls include the reduction of concentrations of inflammatory cytokine TNF-α. The adipokine leptin is reduced in obese animals, increased in obesity and it can contribute to the inflammatory process. However in control animals, the strain induces leptin synthesis which contributes to reducing consumption, increasing energy expenditure and lipid oxidation and preventing overweight and obesity.

### 3.5. Evolution of the effect on the concentration of inflammatory cytokines in the brain

The strain object of the invention also significantly reduces TNF-α synthesis in the central nervous system, the synthesis of which is increased in obesity and contributes to the development of insulin and leptin resistance, inhibiting their anorexigenic effects (reduction of the feeling of hunger) and their function in the regulation of body weight and glucose metabolism (De Souza et al., 2005. Endocrinology., 146: 4192-9).

### 3.6. Evaluation of the effect on the composition of intestinal microbiota and the inflammatory properties thereof

The CECT 7765 strain restores the composition of intestinal microbiota, normalizing the alterations associated with overweight and/or obesity and the inflammatory effect causing these alterations, as well as the alterations associated with other pathological conditions not only associated with overweight and/or obesity. The administration of the strain of the invention increases the number of lactobacilli and bifidobacteria and reduces the number of enterobacteria in the intestinal content by at least half a logarithmic unit. These changes in the composition of the microbiota additionally translate into a reduction of the proinflammatory properties and into an increase of the anti-inflammatory properties thereof. Both in macrophages and in dendritic cells, the microbiota of obese animals that were administered the strain induces lower proinflammatory cytokine synthesis, such as TNF-α synthesis, and greater anti-inflammatory cytokine IL-10 syntheses than that of obese animals that have not been administered the strain (Example 3; Figures 2 and 4). Alterations of intestinal microbiota are considered one of the possible inflammatory stimuli causing weight gain, insulin resistance, obesity and diabetes (Cani and Delzenne 2009. Curr Opin Pharmacol., 9(6): 737-43), said alterations further cause pathological conditions of another type.

**Table 3. Example of the effect of the administration of the strain on the composition of intestinal microbiota of obese animals**

| Bacterial group | Obese (n=6) | | Obese+ CECT 7765 | | ²P-value |
|---|---|---|---|---|---|
| | ¹Median | IQR | ¹Median | IQR | |
| Total bacteria | 8.9 | 8.0-9.2 | 9.0 | 8.8-9.1 | 0.855 |
| *Lactobacillus* | 7.9 | 7.7-8.0 | 8.3 | 8.1-8.6 | 0.004* |
| *Bifidobacterium* | 5.4 | 5.2-5.8 | 8.2 | 7.8-8.7 | 0.011* |
| *Enterobacteriaceae* | 6.6 | 6.3-6.7 | 6.0 | 5.8-6.4 | 0.035* |

| | | | | | |
|---|---|---|---|---|---|
| ¹Data expressed in log no. of copies of the 16S rRNA gene / mg of feces (median, interquartile). ²Statistically significant differences established at a p-value <0.050 applying the Mann-Whitney test. | | | | | |

### Example 4. Effect of the administration of the B. pseudocatenulatum CECT 7765 strain on liver and adipose tissue

The same mice described in Example 3 and the same experimental groups, two of which were administered the strain object of the invention following the same regimen were used. After treatment time, the animals were anesthetized and sacrificed by cervical dislocation and adipose tissue (epididymal) and hepatic tissue samples were taken, which were washed with saline solution and fixed in a buffer with 10% formalin, embedded in paraffin and cut into 4-5 µm sections which were stained with hematoxylin eosin. The severity of the steatosis (lipid accumulation in the liver) was determined by analyzing 10 fields of each fixed section with a bright-field optical microscope (Olympus), according to the following scale: grade 1 (without steatosis); grade 2, when the fat of the hepatocytes occupied less than 33% of the cell; grade 3, when the fat of the hepatocytes occupied between 34-66% of the cell; grade 4, when the fat of the hepatocytes occupied more than 66% of the cell. The size of the adipocytes was measured by means of image analysis using NIS Elements BR 2.3 software, evaluating at least 100 cells per each experimental group and tissue type.

The strain object of the invention reduces the size of the adipocytes in the epididymal tissue the increase (hypertrophy) of which in certain stages of life (childhood and adolescence) favors the development of overweight and obesity in adult age and is associated with a positive imbalance between consumption and energy expenditure (Macia et al., 2006. Genes Nutr., 1: 189-212). In contrast, the reduction in the size of the adipocytes is related with the reduction of insulin resistance and of the concentrations of glucose (Varady et al., 2009. Metabolism 58: 1096-101). In particular, the administration of the strain object of the invention *in vivo* to animal models of obesity leads to an increase of small-sized adipocytes, in the range between 1,000 and 2000 µm², whereas in obese animals that were not administered the strain, large-sized adipocytes are increased in the range between 4000 and 6000 µm² (Figure 5). A similar effect is observed in non-obese animals.

The increased size of the adipocytes is also related with the increased supply of fatty acids to the liver, which leads to hepatic steatosis and its complications, such that the strain can likewise contribute to preventing or improving these alterations. Therefore, the *B. pseudocatenulatum* CECT 7765 strain reduces the size of the adipocytes, i.e., it is useful for the treatment of alterations in the development of cells of this type which leads to their hypertrophy which, maintained over time, can cause overweight and obesity, as well as other pathologies not necessarily associated with obesity.

The strain object of the invention reduces fat accumulation in the liver (steatosis) associated with the consumption of high-fat diets, with obesity and with different pathologies such as non-alcoholic hepatitis (Musso et al., 2010. Hepatology 52: 79-104). In particular, the administration of the strain object of the invention *in vivo* to animal models of obesity leads to a reduction of grade 4 hepatocytes with high fat accumulation (occupying more than 66% of the cell), and an increase of grade 3 hepatocytes with less fat content (occupying 34-66% of a cell), whereas in obese animals that were not administered the strain the effect is the opposite. In control animals, the administration of the strain increases grade 2 hepatocytes (the fat occupies less than 33% of the hepatocyte) at the expense of grade 3, the opposite of what happens in animals that have not received the strain (Figure 6).

### Example 5. Effect of the administration of the B. pseudocatenulatum CECT 7765 strain on peripheral blood concentrations of glucose, insulin, triglycerides and cholesterol

The same mice described in Example 3 and the same experimental groups, two of which were administered the strain of the invention following the same regimen were used. After treatment time, the animals were anesthetized and sacrificed by cervical dislocation and peripheral blood samples were taken for determining the concentration of glucose, triglycerides and cholesterol by means of colorimetric methods (Quimica Clinica Applicada, SA, Amposta, Spain) and the concentration of insulin was determined by ELISA (*BD Bioscience, San Diego, CA, USA*). Furthermore, the postprandial glycemic response or glucose tolerance was determined before sacrificing the animals. After a fasting period, the concentration of basal glucose was measured and then a glucose load of 2 grams per Kg of weight was administered to each mouse and glucose was measured every 15 or 30 minutes with reactive strips (Ascensia Esyfill, Bayer) and using the corresponding glucose meter (Ascensia BRIO, Bayer), with a limit of detection between 30 and 550 mg/dl.

The strain of the invention administered *in vivo* regulates glucose metabolism by reducing its peripheral blood concentration in obese fasting animals; for example, high serum concentrations of glucose of 492.7 (SD 18.3) mg/dl detected in obese mice tend to normalize by means of the administration of the strain object of the invention, reaching values of 316.5 (SD 20.5) mg/dl, proportional to the reduction in the concentration of insulin. Furthermore, the administration of the strain object of the invention to obese animals reduced the postprandial glucose maximum (411.7 [SD 49.9] versus 352.0 [25.8]) and reduced the area under the curve of glucose (5.422 versus 4.779 cm²). The increase in the plasma concentration of glucose and the persistence of high concentrations after an oral dose is indicative of an alteration in insulin synthesis or response to insulin or other causes, and it can be positively regulated by the strain object of the invention, reducing the risk of developing insulin resistance and diabetes and improving treatment.

The strain of the invention administered *in vivo* regulates the lipid metabolism reducing in particular the peripheral blood concentrations of triglycerides and cholesterol in obese animals; for example the high serum concentrations of triglycerides of 196.0 (SD 14.3) mg/dl detected in obese mice are significantly reduced by means of the administration of the strain object of the invention, reaching values of 147.5 (SD 12.5) mg/dl Likewise, the high serum concentrations of cholesterol in obese animals of 146.9 (SD 12.7) mg/dl are significantly reduced by means of the administration of the strain object of the invention, reaching values of 94.4 (SD 5.7) mg/dl.

### Example 6. Effect of the administration of the B. pseudocatenulatum CECT 7765 strain on the absorption of lipid from the diet in the intestine

The same model of obesity described in Example 3 and the same experimental groups, two of which were administered the strain object of the invention following the same regimen were used. After treatment time, the animals were anesthetized and sacrificed by cervical dislocation and intestinal tissue samples were taken which were washed with saline solution and fixed in a buffer with 10% formalin, embedded in paraffin, and cut into 4-5 µm sections which were stained with hematoxylin eosin. The number of chylomicrons per enterocyte was determined by counting 10 fields of each fixed section with the bright-field optical microscope (Olympus) and was expressed in the number of chylomicrons per enterocyte. As can be seen in Figure 7, the strain of the invention reduces the number of chylomicrons which are formed in the enterocytes by more than 50%. These results are consistent with those of Example 5, which show that the strain of the invention reduces the blood triglyceride concentration.

## Claims

1. *Bifidobacterium pseudocatenulatum* strain with accession number CECT 7765.

2. A strain derived from the strain according to claim 1.

3. The strain according to claim 2, wherein said strain is a genetically modified mutant.

4. The strain according to any of claims 1 to 3, wherein said strain is in the form of viable cells.

5. The strain according to any of claims 1 to 3, wherein said strain is in the form of non-viable cells.

6. A microorganism combination comprising the strain according to any of claims 1 to 5.

7. The microorganism combination according to claim 6, further comprising at least another microorganism.

8. The microorganism combination according to claim 7, wherein the other microorganism is an intestinal bacterium or a lactic bacterium.

9. Cell components, metabolites, secreted molecules or any combinations thereof, obtained from the strain according to any of claims 1 to 5, or from the microorganism combination according to any of claims 6 to 8.

10. A composition comprising the strain according to any of claims 1 to 5; or the microorganism combination according to any of claims 6 to 8; or the cell components, metabolites, secreted molecules, or any combinations thereof, according to claim 9.

11. The composition according to claim 10, wherein said composition is a pharmaceutical composition.

12. The composition according to claim 11, wherein it further comprises at least one pharmaceutically acceptable carrier and/or excipient.

13. The composition according to any of claim 11 or 12, wherein it further comprises another active substance.

14. The composition according to any of claims 11 to 13, wherein said composition is presented in a form suitable for oral, sublingual, nasal, intrathecal, bronchial, lymphatic, rectal, transdermal, inhaled or parenteral administration.

15. The composition according to claim 14, wherein said composition is presented in a form suitable for oral administration.

16. The composition according to claim 10, wherein said composition is a nutritive composition.

17. The composition according to claim 16, wherein said composition is a food, a nutraceutical, a supplement, a probiotic or a symbiotic.

18. The composition according to any of claim 16 or 17, wherein said food is selected from the list comprising: dairy product, plant product, meat product, a snack, chocolate, beverage or baby food.

19. The composition according to any of claims 10 to 18, wherein said composition has a concentration of the strain of between 10³ and 10¹⁴ colony forming units (cfu) per gram or milliliter of final composition.

20. Use of a strain of the *B. pseudocatenulatum* species for the production of a pharmaceutical composition, of a medicinal product or of a nutritive composition.

21. Use of the strain according to any of claims 1 to 5; or of the microorganism combination according to any of claims 6 to 8; or of the cell components, metabolites, secreted molecules, or any combinations thereof, according to claim 9; or of the composition according to claim 10, for the production of a pharmaceutical composition, of a medicinal product or of a nutritive composition.

22. Use according to any of claims 20 or 21, wherein the medicinal product or the nutritive composition is used for the prevention and/or treatment of overweight and/or obesity.

23. Use according to any of claims 20 or 21, wherein the medicinal product or the nutritive composition is used for the prevention and/or treatment of hyperglycemia and/or diabetes.

24. Use according to claim 22, wherein the medicinal product or the nutritive composition is used for the prevention and/or treatment of type 2 diabetes mellitus.

25. Use according to any of claim 20 or 21, wherein the medicinal product or the nutritive composition is used for the treatment and/or prevention of hepatic steatosis or fatty liver.

26. Use according to any of claim 20 or 21, wherein the medicinal product or pharmaceutical composition is used for the treatment and/or prevention of dyslipidemia.

27. Use according to claim 26, wherein dyslipidemia is hypertriglyceridemia.

28. Use according to claim 26, wherein dyslipidemia is hypercholesterolemia.

29. Use according to any of claim 20 or 21, wherein the medicinal product or pharmaceutical composition is used for the treatment and/or prevention of metabolic syndrome.

30. Use according to any of claim 20 or 21, wherein the medicinal product or the nutritive composition is used for improving the function of the immune system of an overweight subject or of an obese subject with respect to an untreated subject.

31. Use according to claim 30, wherein the medicinal product or the nutritive composition is used for the prevention and/or treatment of infections in obese or overweight subjects.

32. Use according to any of claim 20 or 21, wherein the medicinal product or the nutritive composition is used for the prevention and/or treatment of adipocyte hypertrophy.

33. Use according to any of claim 20 or 21, wherein the medicinal product or the nutritive composition is used for reducing intake and increasing the energy expenditure in a non-obese subject with respect to an untreated control subject.

34. Use according to any of claim 20 or 21, wherein the medicinal product or the nutritive composition is used for reducing the synthesis of proinflammatory proteins at the peripheral and central level associated with the development of overweight, obesity, and related pathologies.

35. Use according to any of claim 20 or 21, wherein the medicinal product or the nutritive composition is used for reducing the concentration of enterobacteria in the intestinal content with respect to an untreated control.

36. Use according to claim 35, wherein the reduction in the concentration of enterobacteria in the intestinal content is carried out in an overweight or obese subject.


SEQUENCE LISTING

<110> Consejo Superior de Investigaciones Científicas (C.S.I.C.)

<120> BIFIDOBACTERIUM CECT 7765 Y SU USO EN LA PREVENCIÓN Y/O
TRATAMIENTO DEL SOBREPESO, LA OBESIDAD Y PATOLOGÍAS ASOCIADAS

<130> P7858PC00

<150> ES P201031811
<151> 2010-12-07

<160> 7

<170> PatentIn version 3.5

<210> 1
<211> 1280
<212> DNA
<213> Bifidobacterium pseudocatenulatum CECT 7765

<400> 1
gcttggtggt gagagtggcg aacgggtgag taatgcgtga ccgacctgcc ccatacaccg 60

gaatagctcc tggaaacggg tggtaatgcc ggatgctccg actcctcgca tggggtgtcg 120

ggaaagattt catcggtatg ggatggggtc gcgtcctatc aggtagtcgg cggggtaacg 180

gcccaccgag cctacgacgg gtagccggcc tgagagggcg accggccaca ttgggactga 240

gatacggccc agactcctac gggaggcagc agtggggaat attgcacaat gggcgcaagc 300

ctgatgcagc gacgccgcgt gcgggatgac ggccttcggg ttgtaaaccg cttttgatcg 360

ggagcaagcc ttcgggtgag tgtacctttc gaataagcac cggctaacta cgtgccagca 420

gccgcggtaa tacgtagggt gcaagcgtta tccggaatta ttgggcgtaa agggctcgta 480

ggcggttcgt cgcgtccggt gtgaaagtcc atcgcttaac ggtggatctg cgccgggtac 540

gggcgggctg gagtgcggta ggggagactg gaattcccgg tgtaacggtg gaatgtgtag 600

atatcgggaa gaacaccaat ggcgaagtca ggtctctggg ccgttactga cgctgaggag 660

cgaaagcgtg gggagcgaac aggattagat accctggtag tccacgccgt aaacggtgga 720

tgctggttgt ggggcccgtt ccacgggttc cgtgtcggac ctaacgcgtt aagcatcccg 780

cctggggagt acggccgcaa ggctaaaact caaagaaatt gacgggggcc cgcacaagcg 840

gcggagcatg cggattaatt cgatgcaacg cgaagaacct tacctgggct tgacatgttc 900

ccgacagccg tagagatatg gcctcccttc ggggcgggtt cacaggtggt gcatggtcgt 960

cgtcagctcg tgtcgtgaga tgttgggtta agtcccgcaa cgagcgcaac cctcgccctg 1020

tgttgccagc acgtcatggt gggaactcac gggggaccgc cggggtcaac tcggaggaag 1080

gtggggatga cgtcagatca tcatgcccct tacgtccagg gcttcacgca tgctacaatg 1140

gccggtacaa cgggatgcga cacggcgacg tggagcggat ccctgaaaac cggtctcagt 1200

tcggattgga gtctgcaacc cgactccatg aaggcggagt cgctagtaat cgcggatcag 1260

caacgccgcg gtgaatgcgt                                                    1280

<210>  2
<211>  20
<212>  DNA
<213>  Artificial

<220>
<223>  Cebador 27f

<400>  2
agagtttgat cctggctcag                                                    20

<210>  3
<211>  17
<212>  DNA
<213>  Artificial

<220>
<223>  Cebador 1401r

<400>  3
cggtgtgtac aagaccc                                                       17

<210>  4
<211>  16
<212>  DNA
<213>  Artificial

<220>
<223>  Cebador 530f

<400>  4
gtgccagcag ccgcgg                                                        16

<210>  5
<211>  17
<212>  DNA
<213>  Artificial

<220>
<223>  Cebador U-968f

<400>  5
aacgcgaaga accttac                                                       17

<210>  6
<211>  15
<212>  DNA
<213>  Artificial

<220>
<223>  Cebador M13

<400>  6
gagggtggcg gttct                                                         15

<210> 7
<211> 10
<212> DNA
<213> Artificial

<220>
<223> Cebador Del

<400> 7
ccgcagccaa 10